# EUROPEAN PATENT APPLICATION

(11) **EP 4 711 367 A1**
(43) Date of publication of application: **18.03.2026**
(21) Application number: 24806519.5
(22) Date of filing: 11.05.2024
(51) Int. Cl.: C07D 487/04, C07D 403/14, A61K 31/4985, A61P 29/00, A61P 17/06

(54) **PYRAZOLOPYRAZINE COMPOUND AND METHOD FOR PREPARING CRYSTAL FORM THEREOF**

(30) Priority: 12.05.2023 CN 202310544690
(71) Applicant: CMS Research & Development Pte. Ltd., Singapore 179803 (SG)
(72) Inventor: XIA, Jianhua, Shanghai 200131 (CN); HE, Haiying, Shanghai 200131 (CN); JIANG, Zhigan, Shanghai 200131 (CN)
(74) Representative: Haseltine Lake Kempner LLP
(86) International application number: PCT/CN2024/092691
(87) International publication number: WO 2024/235167

(57) **Abstract**

Disclosed in the present invention are a pyrazolopyrazine compound and a method for preparing a crystal form thereof, and particularly disclosed are a compound of formula (I) and a method for preparing a crystal form thereof.

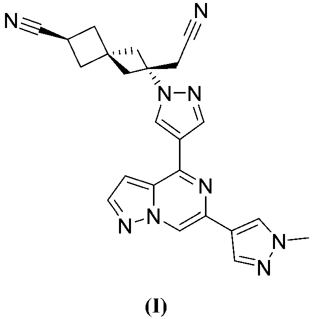

## Description

The present application claims the following priority:

The present application claims the priority and right of the Chinese patent application No. 2023105446906 filed with the China National Intellectual Property Administration on May 12, 2023. The contents of the Chinese patent application are incorporated herein by reference in their entirety.

### TECHNICAL FIELD

The present disclosure relates to a pyrazolopyrazine compound and a method for preparing a crystal form thereof, in particular to a method for preparing a compound of formula (I) and a crystal form thereof.

### BACKGROUND

Janus kinases (JAKs) are cytoplasmic tyrosine kinases that can transmit cytokine signals from membrane receptors to STAT transcription factors. The JAK family comprises four members: JAK1, JAK2, JAK3, and TYK2. The JAK-STAT pathway transmits extracellular signals from various cytokines, growth factors, and hormones into the nucleus and is responsible for the expression of thousands of protein-coding genes. The JAK-STAT pathway converts extracellular signals into transcriptional responses through several steps: 1) When the cytokine receptors on the cell surface bind to their respective cytokine ligands, the conformation changes, causing receptor dimerization. This brings the receptor-coupled JAK kinases into close proximity, leading to their activation via reciprocal tyrosine phosphorylation. 2) Activated JAKs catalyze the phosphorylation of tyrosine residues on the receptor. These phosphorylated tyrosine sites, along with surrounding amino acid sequences, form "docking sites", to which STAT proteins containing SH2 domains are recruited. 3) Finally, the kinase JAK catalyzes the phosphorylation of STAT proteins bound to the receptor. The activated STAT proteins dissociate from the receptor, form dimers, and then translocate into the nucleus to regulate the transcription of specific genes. The JAK-STAT intracellular signaling pathway serves interferons, most interleukins, and various cytokines and endocrine factors, such as EPO, TPO, GH, OSM, LIF, CNTF, GM-CSF, and PRL (Vainchenker W.E T al. 2008).

Different members of the JAK family selectively bind to distinct cytokine receptors, conferring signaling specificity and thereby exerting diverse physiological effects. This selective mode of action allows JAK inhibitors to be applied with relative specificity in disease treatment. TYK2 is a member of the JAK kinase family and serves as a key mediator in the signaling of inflammatory cytokines such as IL-12, IL-23, and type I interferons. These cytokines are related to the pathogenesis of various inflammatory and autoimmune diseases, including psoriasis, inflammatory bowel disease (IBD), and systemic lupus erythematosus (SLE). Therefore, developing inhibitors with high inhibitory activity against TYK2 and certain inhibitory activity against JAK2 and JAK1 can maximally suppress the formation of heterodimers between TYK2 and JAK2 or JAK1 within cells, block the signaling pathways, and consequently interrupt the signal transduction of inflammatory cytokines IL-12, IL-23, and type I interferons, thereby treating specific diseases.

### SUMMARY

The present disclosure provides a method for preparing a compound of formula (I), 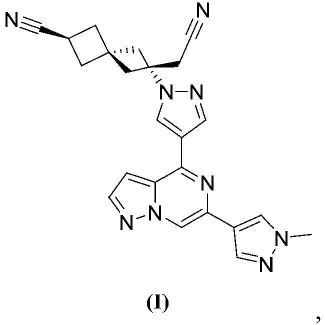 wherein the method comprises a chiral resolution method for compound q, 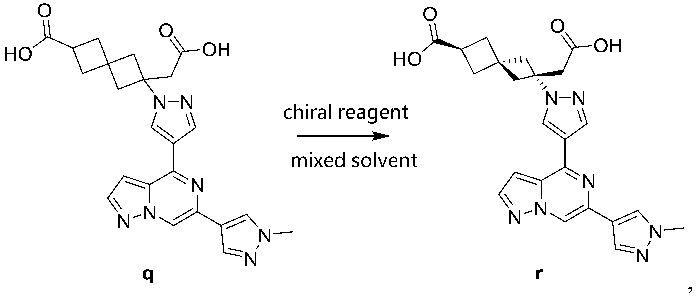 wherein
the chiral reagent is selected from the group consisting of chiral reagent 1 and chiral reagent 2;
the chiral reagent 1 is (*R*)-(+)-*N*-benzyl-1-phenylethylamine, and the chiral reagent 2 is (*S*)-(-)-*N*-benzyl-1-phenylethylamine;
the mixed solvent is selected from the group consisting of solvent 2 and solvent 3;
the solvent 2 is tetrahydrofuran, and the solvent 3 is water.

In some embodiments of the present disclosure, the molar ratio of compound q to the chiral reagent is 1:2 to 1:2.5, preferably 1:2.1, and other variables are as defined in the present disclosure.

In some embodiments of the present disclosure, the volume ratio of the mixed solvent is tetrahydrofuran: water = 15:1 to 20:1; preferably 16.5:1, and other variables are as defined in the present disclosure.

In some embodiments of the present disclosure, the chiral resolution method for compound q further comprises a step of further purification, and other variables are as defined in the present disclosure.

In some embodiments of the present disclosure, the chiral resolution method for compound q and the purification step are: at 20 to 30°C, sequentially adding solvent 2, solvent 3, compound q, and chiral reagent 1 into a reactor, stirring at 20 to 30 °C for 1 hour, raising the temperature to 73°C and stirring for 2 hours, naturally cooling to 20 to 30 °C, and stirring at this temperature for 12 hours, filtering the reaction mixture, and washing the filter cake three times with a mixed solvent of solvent 2 and solvent 3. Concentrating the filtrate under reduced pressure, slurrying with an aqueous sodium carbonate solution for 1 hour, adding ethyl acetate, stirring for another 1 hour, allowing phase separation by standing, washing the aqueous phase with ethyl acetate, and discarding the organic phases from both washes. Transferring the aqueous phase to a reactor, adding solvent 15, at which point the solvent in the reactor is water and solvent 15, adjusting the pH to 4 to 5 with concentrated hydrochloric acid, stirring at 20 to 30°C for 12 hours to generate a solid, filtering the reaction mixture, and vacuum drying the resulting filter cake at 70°C for 12 hours to obtain crude compound r. At 20 to 30°C, sequentially adding solvent 2, solvent 3, the above crude compound r, and chiral reagent 2 into a reactor, stirring at 20 to 30°C for 1 hour, raising the temperature to 73°C and stirring for 2 hours, naturally cooling to 20 to 30°C, and stirring at this temperature for 12 hours, filtering the reaction mixture, and washing the filter cake three times with a mixed solvent of solvent 2 and solvent 3. At 20 to 30°C, adding solvent 2, solvent 3, and the above filter cake sequentially into a reactor, stirring at 20 to 30°C for 1 hour, raising the temperature to 73°C, stirring for 2 hours, naturally cooling 20 to 30°C, stirring at this temperature for 12 hours, filtering the reaction mixture, and washing the filter cake three times with a mixed solvent of solvent 2 and solvent 3. Slurrying the filter cake with an aqueous sodium carbonate solution for 1 hour, adding ethyl acetate, stirring for another 1 hour, allowing phase separation by standing, washing the aqueous phase again with ethyl acetate, and discarding the organic phases from both washes. Transferring the aqueous phase to a reactor, adding acetonitrile, at which point the solvent in the reactor is water and solvent 15, adjusting the pH to 4 to 5 with concentrated hydrochloric acid, stirring at 20 to 30°C for 12 hours, forming a solid, filtering the reaction mixture, and vacuum drying the filter cake at 70°C for 12 hours to obtain compound r. SFC analysis method (column type: Chiralcel OJ-3 100 × 4.6 mm I.D., 3 µm; mobile phase: A: CO₂; B: methanol (0.05% diethylamine); B-phase gradient: 5%-40% for 4 min, maintaining 40% for 2.5 min, 5% for 1.5 min; flow rate: 2.8 mL/min); retention time: 3.285 minutes; ee% = 99.02%.

In some embodiments of the present disclosure, the solvent 2 is tetrahydrofuran, the solvent 3 is water, the solvent 15 is acetonitrile, the chiral reagent 1 is (*R*)-(+)-*N*-benzyl-1-phenylethylamine, the chiral reagent 2 is (*S*)-(-)-*N*-benzyl-1-phenylethylamine, and other variables are as defined in the present disclosure.

In some embodiments of the present disclosure, the volume ratio of the solvent 2 to the solvent 3 is 15:1 to 20:1, preferably 16.5:1, and other variables are as defined in the present disclosure.

In some embodiments of the present disclosure, the volume ratio of water to the solvent 15 is 5:1 to 15:1, preferably 10:1, and other variables are as defined in the present disclosure.

In some embodiments of the present disclosure, the molar ratio of compound q to the chiral reagent 1 is 1:2 to 1:2.5, preferably 1:2.1, and other variables are as defined in the present disclosure.

In some embodiments of the present disclosure, the molar ratio of crude compound r to the chiral reagent 2 is 1:2 to 1:2.5, preferably 1:2.1, and other variables are as defined in the present disclosure.

The present disclosure provides a method for preparing the compound of formula (I), wherein the method comprises performing a selective alkylation method between compound d and compound e, 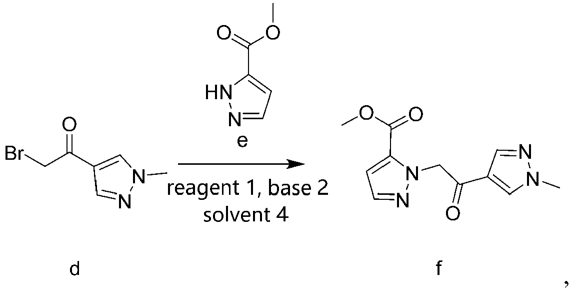 wherein
the reagent 1 is magnesium bromide;
the base 2 is DIEA;
the solvent 4 is tetrahydrofuran.

In some embodiments of the present disclosure, the step of performing a selective alkylation method between compound d and compound e comprises: dissolving compound e and compound d in the solvent 4, adding the reagent 1 after dissolving completely, slowly adding the base 2 dropwise at 15 to 20°C, and continuing the reaction while maintaining the temperature to obtain compound f.

In some embodiments of the present disclosure, the reagent 1 is magnesium bromide, the base 2 is DIEA, the solvent 4 is tetrahydrofuran, and other variables are as defined in the present disclosure.

In some embodiments of the present disclosure, the molar ratio of compound e to compound d is 1:0.8 to 1:1.2, preferably 1:1, and other variables are as defined in the present disclosure.

In some embodiments of the present disclosure, the molar ratio of compound e, compound d, and the reagent 1 is 1:0.8:0.1 to 1:1.2:0.3, preferably 1:1:0.2, and other variables are as defined in the present disclosure.

In some embodiments of the present disclosure, the time for adding the base 2 dropwise is 2 to 4 hours, preferably 3 hours, and other variables are as defined in the present disclosure.

In some embodiments of the present disclosure, the reaction time is 10 to 20 hours, preferably 15 hours, and other variables are as defined in the present disclosure.

The present disclosure provides a method for preparing the compound of formula (I), comprising the following reaction route:

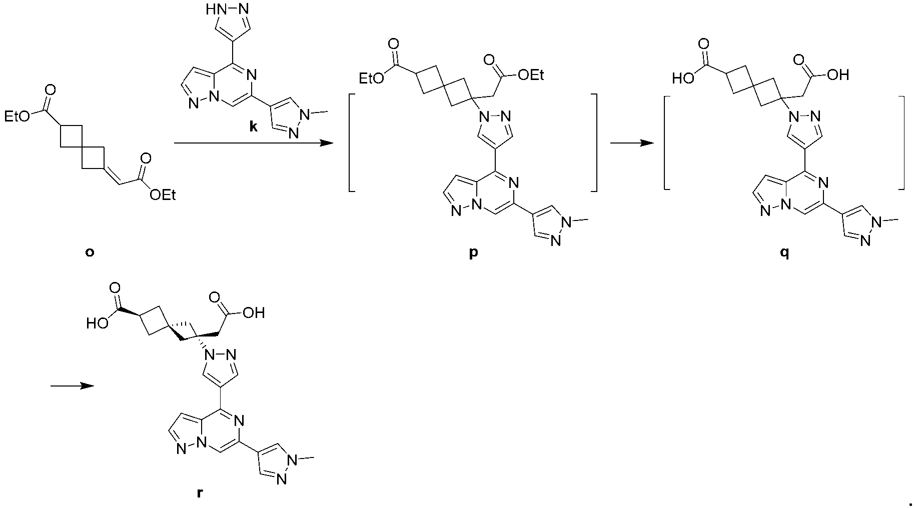

The present disclosure provides a method for preparing the compound of formula (I), comprising a method for synthesizing compound r from compound o, 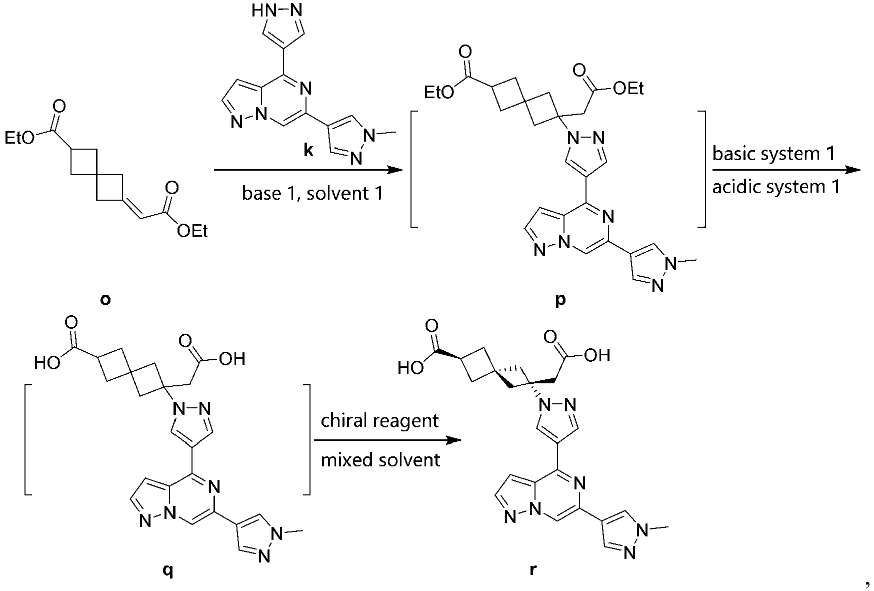
the equivalent ratio of compound o to compound k is 1:0.9 to 1:1.2;
the base 1 is DBU;
the solvent 1 is acetonitrile;
the basic system 1 is an aqueous sodium hydroxide solution;
the acidic system 1 is concentrated hydrochloric acid;
the chiral reagent is selected from the group consisting of chiral reagent 1 and chiral reagent 2;
the chiral reagent 1 is (*R*)-(+)-*N*-benzyl-1-phenylethylamine, and the chiral reagent 2 is (*S*)-(-)-*N*-benzyl-1-phenylethylamine;
the mixed solvent is selected from the group consisting of solvent 2 and solvent 3;
the solvent 2 is tetrahydrofuran, and the solvent 3 is water.

In some embodiments of the present disclosure, the reaction temperature for preparing compound p from compound o and compound k is 85 to 95°C, preferably the internal temperature is 82°C, and other variables are as defined in the present disclosure.

In some embodiments of the present disclosure, the equivalent ratio of compound o to compound k is 1:1, and other variables are as defined in the present disclosure.

The present disclosure provides a method for preparing the compound of formula (I), comprising the following reaction route:

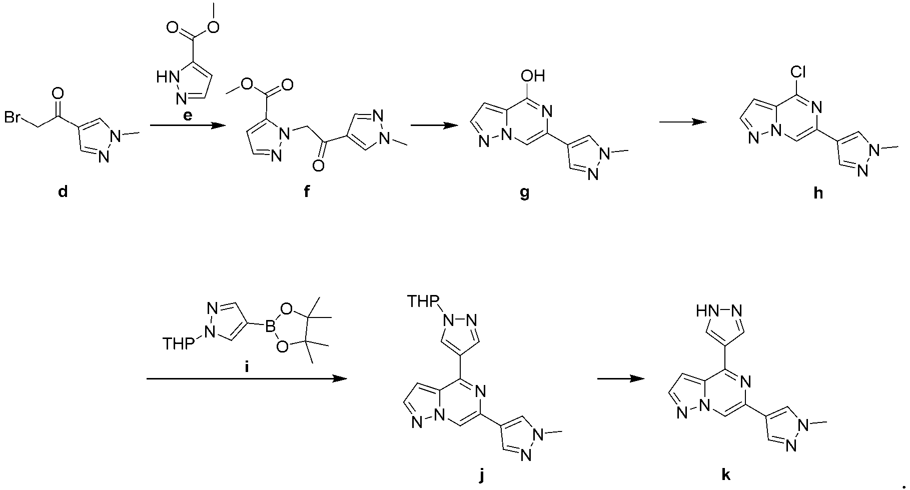

The present disclosure provides a method for preparing the compound of formula (I), comprising the following reaction route:

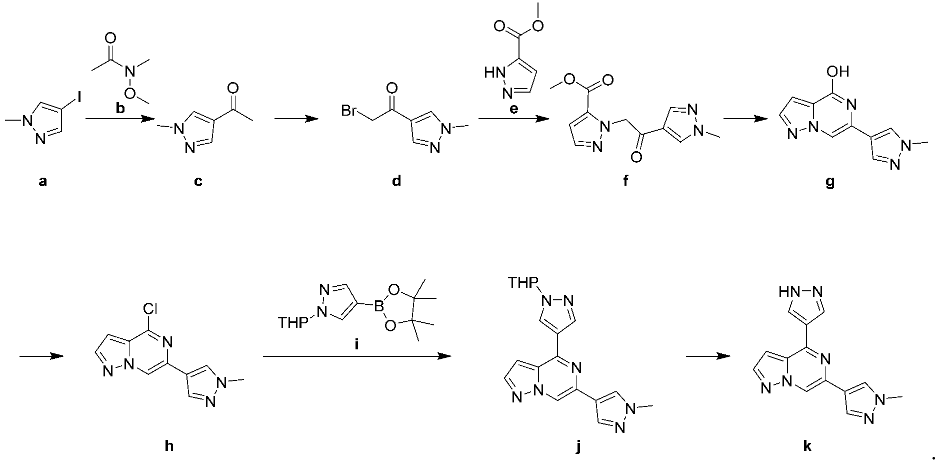

The present disclosure provides a method for preparing the compound of formula (I), wherein the method comprises a method for synthesizing intermediate k, 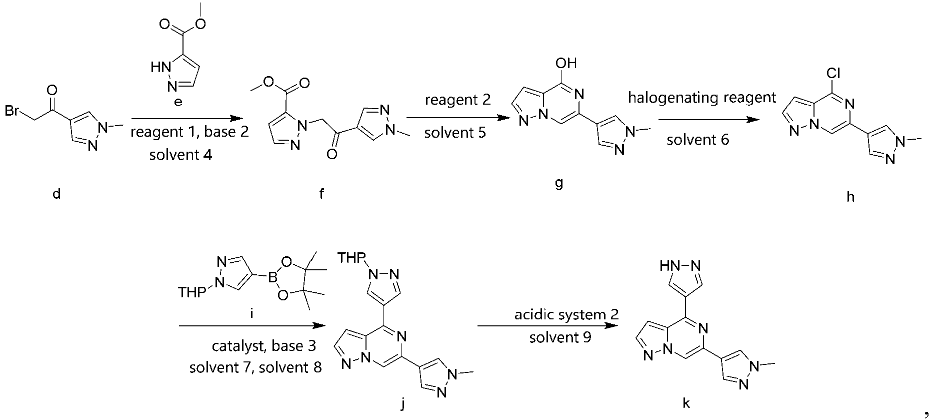
the reagent 1 is magnesium bromide;
the base 2 is DIEA;
the solvent 4 is tetrahydrofuran;
the reagent 2 is ammonium acetate;
the solvent 5 is ethanol;
the halogenating reagent is phosphorus oxychloride;
the solvent 6 is acetonitrile;
the catalyst is [1,1'-bis(diphenylphosphino)ferrocene]dichloropalladium(II);
the base 3 is sodium carbonate;
the solvent 7 is dioxane;
the solvent 8 is water;
the acidic system 2 is a hydrogen chloride/ethyl acetate solution;
the solvent 9 is dichloromethane.

In some embodiments of the present disclosure, the volume ratio of the solvent 7 to the solvent 8 is 4:1 to 6:1, preferably 5:1, and other variables are as defined in the present disclosure.

In some embodiments of the present disclosure, the molar ratio of compound h, compound i, the catalyst, and the base 3 is 1:0.9:0.01:2 to 1:1.2:0.05:3, preferably 1:1.05:0.03:2.5, and other variables are as defined in the present disclosure.

In some embodiments of the present disclosure, the concentration of the hydrogen chloride/ethyl acetate solution is 3 to 5 M, preferably 4 M, and other variables are as defined in the present disclosure.

The present disclosure provides a method for preparing the compound of formula (I), further comprising the following reaction steps:

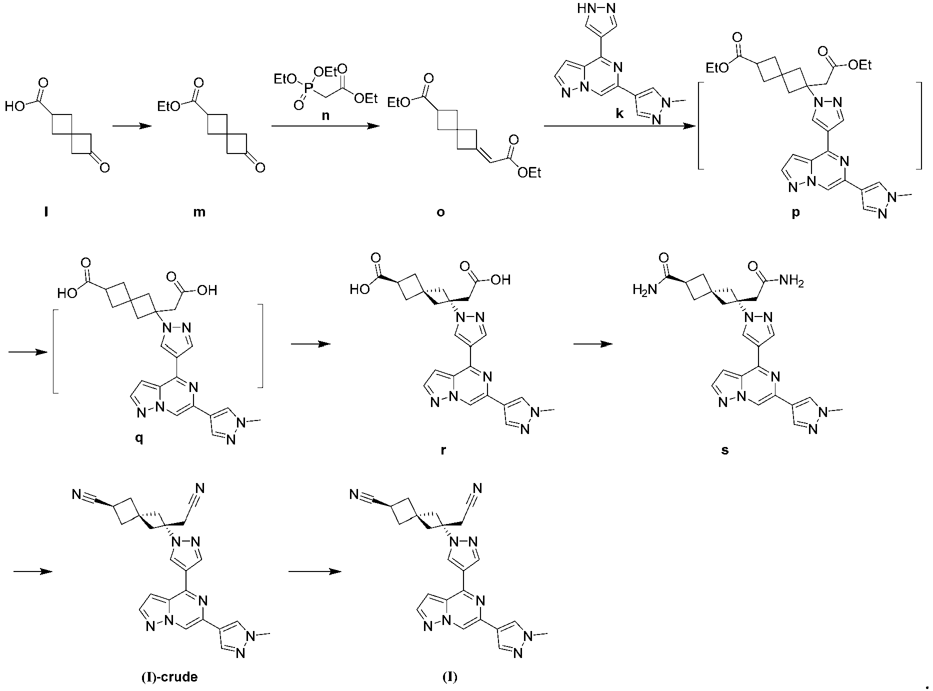

The present disclosure provides a method for preparing the compound of formula (I), further comprising a method for synthesizing the compound of formula (I) from compound 1, 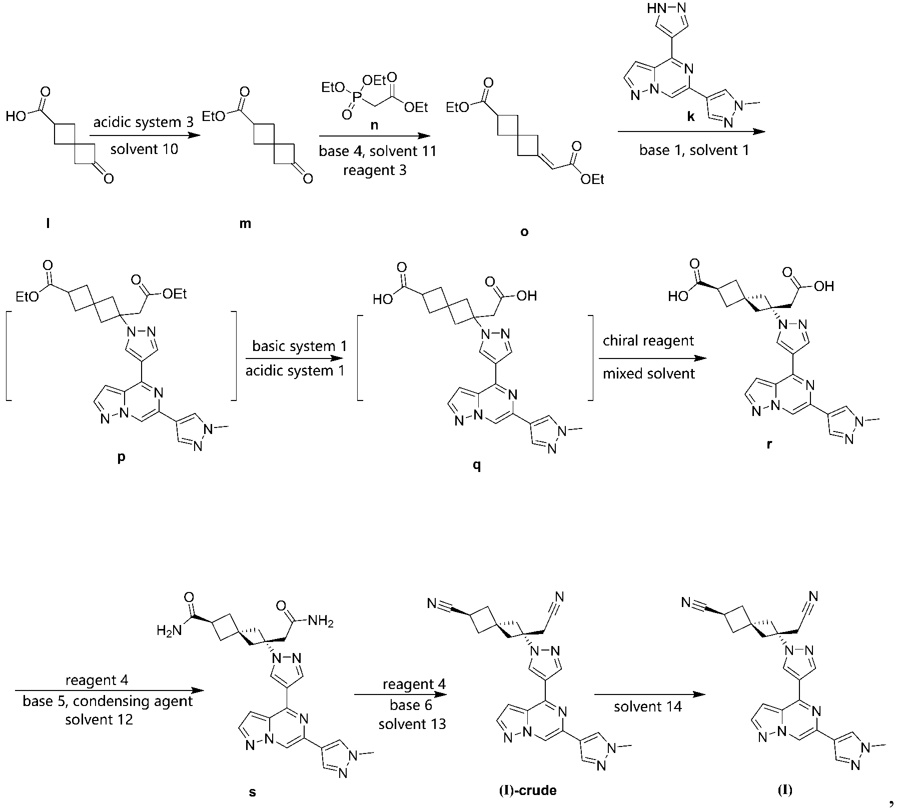
the acidic system 3 is sulfuric acid;
the solvent 10 is ethanol;
the base 4 is triethylamine;
the solvent 11 is tetrahydrofuran;
the reagent 3 is lithium bromide;
the solvent 1 is acetonitrile;
the basic system 1 is an aqueous sodium hydroxide solution;
the acidic system 1 is concentrated hydrochloric acid;
the chiral reagent is selected from the group consisting of chiral reagent 1 and chiral reagent 2;
the chiral reagent 1 is (*R*)-(+)-*N*-benzyl-1-phenylethylamine, and the chiral reagent 2 is (*S*)-(-)-*N*-benzyl-1-phenylethylamine;
the mixed solvent is selected from the group consisting of solvent 2 and solvent 3;
the solvent 2 is tetrahydrofuran, and the solvent 3 is water;
the reagent 4 is ammonium chloride;
the base 5 is DIEA;
the condensing agent is HATU;
the solvent 12 is DMF;
the reagent 4 is trifluoroacetic anhydride;
the base 6 is triethylamine;
the solvent 13 is dichloromethane;
the solvent 14 is methanol.

The present disclosure provides a Form 1 crystal form of a compound of formula (I), 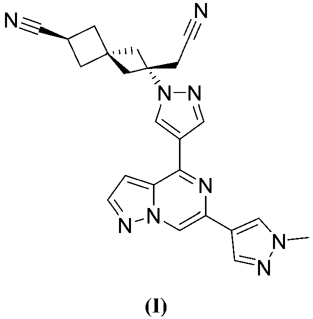 wherein the Form 1 crystal form of the compound of formula (I) has an X-ray powder diffraction pattern, measured using Cu Kα radiation, comprising characteristic diffraction peaks at the following 20 angles: 8.087 ± 0.200°, 9.379 ± 0.200°, 11.188 ± 0.200°, and 12.852 ± 0.200°.

In some embodiments of the present disclosure, the Form 1 crystal form of the compound of formula (I) has an X-ray powder diffraction pattern, expressed in terms of 20 angles, comprising at least 6, 7, 8, or 9 diffraction peaks selected from the group consisting of: 8.087 ± 0.200°, 9.379 ± 0.200°, 11.188 ± 0.200°, 12.852 ± 0.200°, 16.163 ± 0.200°, 18.306 ± 0.200°, 18.626 ± 0.200°, 19.105 ± 0.200°, 19.447 ± 0.200°, and 20.666 ± 0.200°.

In some embodiments of the present disclosure, the Form 1 crystal form of the compound of formula (I) has an X-ray powder diffraction pattern comprising characteristic diffraction peaks at the following 2θ angles: 8.087 ± 0.200°, 9.379 ± 0.200°, 11.188 ± 0.200°, 12.852 ± 0.200°, 16.163 ± 0.200°, 18.306 ± 0.200°, 18.626 ± 0.200°, and 19.105 ± 0.200°.

In some embodiments of the present disclosure, the Form 1 crystal form of the compound of formula (I) has an X-ray powder diffraction pattern, expressed in terms of 20 angles, comprising at least 11, 12, 13, or 14 diffraction peaks selected from the group consisting of: 8.087 ± 0.200°, 9.379 ± 0.200°, 11.188 ± 0.200°, 12.852 ± 0.200°, 16.163 ± 0.200°, 18.306 ± 0.200°, 18.626 ± 0.200°, 19.105 ± 0.200°, 19.447 ± 0.200°, 20.666 ± 0.200°, 20.875 ± 0.200°, 21.210 ± 0.200°, 22.417 ± 0.200°, 23.446 ± 0.200°, and 24.043 ± 0.200°.

In some embodiments of the present disclosure, the Form 1 crystal form of the compound of formula (I) has an X-ray powder diffraction pattern comprising characteristic diffraction peaks at the following 2θ angles: 8.087 ± 0.200°, 9.379 ± 0.200°, 11.188 ± 0.200°, 12.852 ± 0.200°, 16.163 ± 0.200°, 18.306 ± 0.200°, 18.626 ± 0.200°, 19.105 ± 0.200°, 19.447 ± 0.200°, 20.666 ± 0.200°, 20.875 ± 0.200°, and 21.210 ± 0.200°.

In some embodiments of the present disclosure, the Form 1 crystal form of the compound of formula (I) has an X-ray powder diffraction pattern, expressed in terms of 2θ angles, comprising at least 16, 17, 18, 19, 20, 21, 22, or 23 diffraction peaks selected from the group consisting of: 8.087 ± 0.200°, 9.379 ± 0.200°, 11.188 ± 0.200°, 12.852 ± 0.200°, 16.163 ± 0.200°, 18.306 ± 0.200°, 18.626 ± 0.200°, 19.105 ± 0.200°, 19.447 ± 0.200°, 20.666 ± 0.200°, 20.875 ± 0.200°, 21.210 ± 0.200°, 22.417 ± 0.200°, 23.446 ± 0.200°, 24.043 ± 0.200°, 24.402 ± 0.200°, 25.159 ± 0.200°, 26.227 ± 0.200°, 26.742 ± 0.200°, 26.897 ± 0.200°, 27.725 ± 0.200°, 30.956 ± 0.200°, 32.574 ± 0.200°, and 34.479 ± 0.200°.

In some embodiments of the present disclosure, the Form 1 crystal form of the compound of formula (I) has an X-ray powder diffraction pattern comprising characteristic diffraction peaks at the following 2θ angles: 8.087 ± 0.200°, 9.379 ± 0.200°, 11.188 ± 0.200°, 12.852 ± 0.200°, 16.163 ± 0.200°, 18.306 ± 0.200°, 18.626 ± 0.200°, 19.105 ± 0.200°, 19.447 ± 0.200°, 20.666 ± 0.200°, 20.875 ± 0.200°, 21.210 ± 0.200°, 22.417 ± 0.200°, 23.446 ± 0.200°, 24.043 ± 0.200°, and 24.402 ± 0.200°.

In some embodiments of the present disclosure, the Form 1 crystal form of the compound of formula (I) has an X-ray powder diffraction pattern comprising characteristic diffraction peaks at the following 2θ angles: 6.516°, 8.087°, 9.378°, 11.188°, 11.881°, 12.852°, 13.880°, 14.564°, 15.615°, 16.163°, 18.306°, 18.626°, 19.105°, 19.477°, 20.666°, 20.875°, 21.210°, 22.417°, 23.446°, 23.715°, 24.043°, 24.402°, 25.159°, 25.663°, 26.227°, 26.742°, 26.897°, 27.127°, 27.752°, 28.341°, 29.332°, 30.727°, 30.956°, 31.678°, 32.197°, 32.574°, 34.479°, 35.051°, 35.341°, 35.873°, and 36.960°.

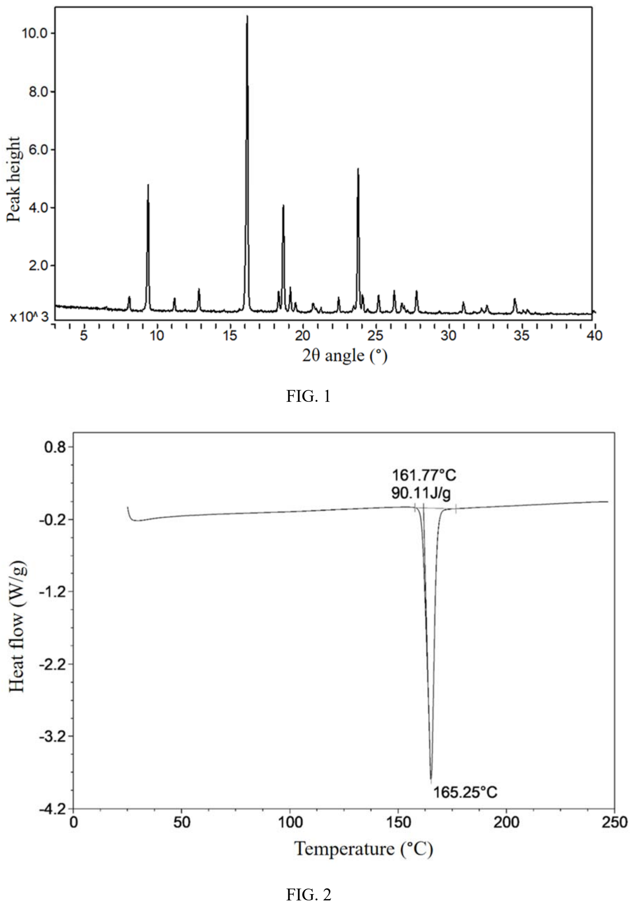

In some embodiments of the present disclosure, the Form 1 crystal form of the compound of formula (I) has an XRPD pattern as shown in FIG. 1.

In some embodiments of the present disclosure, the Form 1 crystal form of the compound of formula (I) has a differential scanning calorimetry (DSC) curve showing an endothermic peak onset at 161.77°C ± 3.00°C.

In some embodiments of the present disclosure, the Form 1 crystal form of the compound of formula (I) has a DSC pattern as shown in FIG. 2.

In some embodiments of the present disclosure, the Form 1 crystal form of the compound of formula (I) has a thermogravimetric analysis (TGA) curve showing a weight loss of 0.3182% at 150.00°C ± 3.00°C.

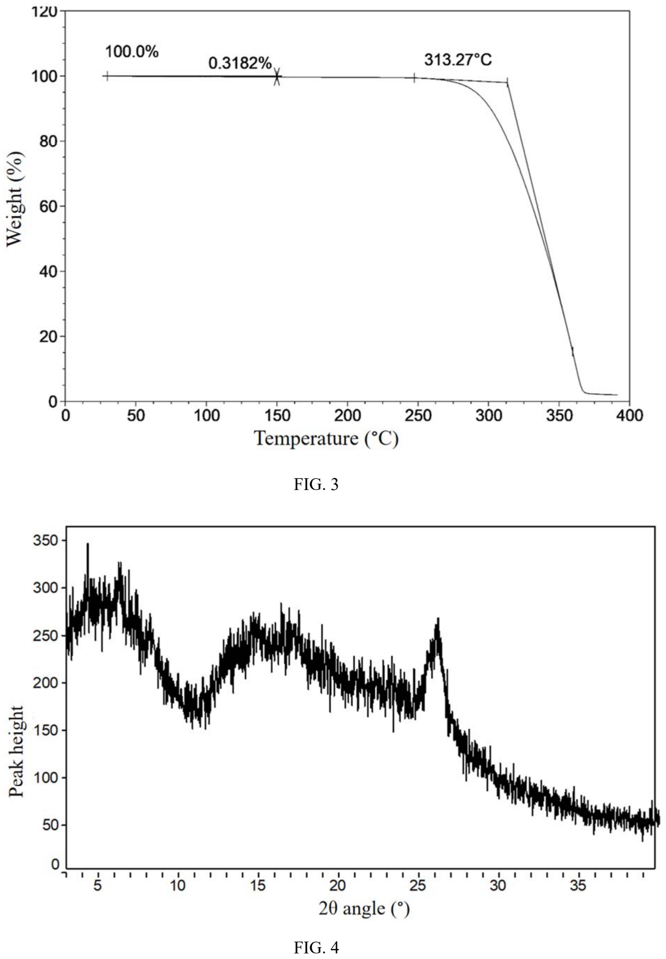

In some embodiments of the present disclosure, the Form 1 crystal form of the compound of formula (I) has a TGA pattern as shown in FIG. 3.

In some embodiments of the present disclosure, the Form 1 crystal form of the compound of formula (I) has an XRPD pattern measured using Cu Kα radiation, wherein the peak positions and relative intensities of the diffraction peaks are as shown in Table 1 below:

**Table 1. XRPD pattern analysis data of the Form 1 crystal form of the compound of formula (I)**

| No. | 2θ angle (°) | *d-*Spacing (Å) | Relative intensity (%) | Peak height | No. | 2θ angle (°) | *d-*Spacing (Å) | Relative intensity (%) | Peak height |
|---|---|---|---|---|---|---|---|---|---|
| 1 | 6.516 | 13.5527 | 0.7 | 73 | 22 | 24.402 | 3.6448 | 1.1 | 111 |
| 2 | 8.087 | 10.9236 | 4.8 | 493 | 23 | 25.159 | 3.5368 | 5.8 | 589 |
| 3 | 9.379 | 9.4217 | 42.9 | 4369 | 24 | 25.663 | 3.4684 | 0.5 | 52 |
| 4 | 11.188 | 7.9018 | 4.4 | 445 | 25 | 26.227 | 3.3951 | 7.5 | 765 |
| 5 | 11.881 | 7.4424 | 0.6 | 64 | 26 | 26.742 | 3.3308 | 3.2 | 326 |
| 6 | 12.852 | 6.8826 | 7.6 | 779 | 27 | 26.897 | 3.3120 | 2.4 | 246 |
| 7 | 13.880 | 6.3751 | 0.5 | 46 | 28 | 27.127 | 3.2845 | 0.8 | 79 |
| 8 | 14.564 | 6.0770 | 0.8 | 81 | 29 | 27.752 | 3.2119 | 7.5 | 763 |
| 9 | 15.615 | 5.6702 | 0.5 | 50 | 30 | 28.341 | 3.1464 | 0.4 | 45 |
| 10 | 16.163 | 5.4791 | 100.0 | 10184 | 31 | 29.332 | 3.0424 | 0.9 | 91 |
| 11 | 18.306 | 4.8424 | 6.3 | 640 | 32 | 30.727 | 2.9074 | 0.6 | 58 |
| 12 | 18.626 | 4.7599 | 35.8 | 3644 | 33 | 30.956 | 2.8864 | 4.0 | 404 |
| 13 | 19.105 | 4.6417 | 8.0 | 812 | 34 | 31.678 | 2.8222 | 0.5 | 56 |
| 14 | 19.477 | 4.5539 | 3.1 | 314 | 35 | 32.197 | 2.7779 | 1.6 | 162 |
| 15 | 20.666 | 4.2943 | 3.2 | 328 | 36 | 32.574 | 2.7466 | 2.8 | 281 |
| 16 | 20.875 | 4.2520 | 1.6 | 158 | 37 | 34.479 | 2.5991 | 5.1 | 517 |
| 17 | 21.210 | 4.1855 | 1.7 | 170 | 38 | 35.051 | 2.5579 | 1.1 | 108 |
| 18 | 22.417 | 3.9627 | 5.1 | 521 | 39 | 35.341 | 2.5376 | 1.3 | 131 |
| 19 | 23.446 | 3.7910 | 2.3 | 235 | 40 | 35.873 | 2.5012 | 0.5 | 53 |
| 20 | 23.751 | 3.7430 | 48.6 | 4951 | 41 | 36.960 | 2.4301 | 0.4 | 41 |
| 21 | 24.043 | 3.6982 | 5.4 | 550 | | | | | |

The present disclosure provides a near-amorphous form of a compound of formula (I), 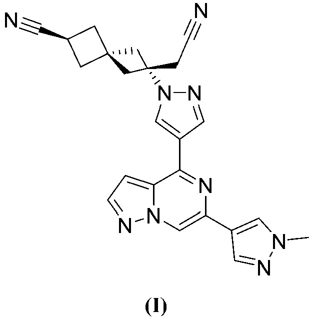 wherein the near-amorphous form of the compound of formula (I) has an X-ray powder diffraction pattern, measured using Cu Kα radiation, comprising characteristic diffraction peaks at the following 2θ angles: 4.341 ± 0.200°, 5.082 ± 0.200°, and 6.921 ± 0.200°.

In some embodiments of the present disclosure, the near-amorphous form of the compound of formula (I) has an X-ray powder diffraction pattern, expressed in terms of 20 angles, comprising at least 4, 5, or 6 diffraction peaks selected from the group consisting of: 4.341 ± 0.200°, 5.082 ± 0.200°, 6.921 ± 0.200°, 18.955 ± 0.200°, 23.319 ± 0.200°, and 26.108 ± 0.200°.

In some embodiments of the present disclosure, the near-amorphous form of the compound of formula (I) has an X-ray powder diffraction pattern comprising characteristic diffraction peaks at the following 2θ angles: 4.341 ± 0.200°, 5.082 ± 0.200°, 6.921 ± 0.200°, and 18.955 ± 0.200°.

In some embodiments of the present disclosure, the near-amorphous form of the compound of formula (I) has an X-ray powder diffraction pattern comprising characteristic diffraction peaks at the following 2θ angles: 4.341 ± 0.200°, 5.082 ± 0.200°, 6.921 ± 0.200°, 18.955 ± 0.200°, and 23.319 ± 0.200°.

In some embodiments of the present disclosure, the amorphous form of the compound of formula (I) has an X-ray powder diffraction pattern comprising characteristic diffraction peaks at the following 2θ angles: 4.341°, 5.082°, 6.921°, 18.955°, 23.319°, and 26.108°.

In some embodiments of the present disclosure, the near-amorphous form of the compound of formula (I) has an XRPD pattern as shown in FIG. 4.

In some embodiments of the present disclosure, the near-amorphous form of the compound of formula (I) has a differential scanning calorimetry (DSC) curve showing an endothermic peak onset at 76.70°C ± 3.00°C.

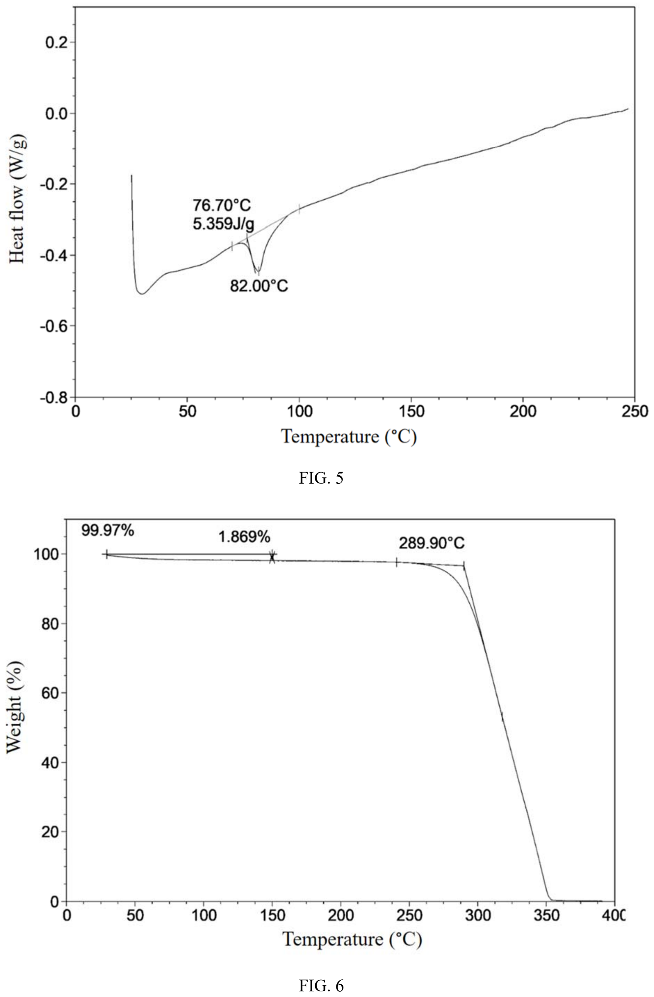

In some embodiments of the present disclosure, the near-amorphous form of the compound of formula (I) has a DSC pattern as shown in FIG. 5.

In some embodiments of the present disclosure, the near-amorphous form of the compound of formula (I) has a thermogravimetric analysis (TGA) curve showing a weight loss of 1.869% at 150.00°C ± 3.00°C.

In some embodiments of the present disclosure, the near-amorphous form of the compound of formula (I) has a TGA pattern as shown in FIG. 6.

In some embodiments of the present disclosure, the near-amorphous form of the compound of formula (I) has an XRPD pattern measured using Cu Kα radiation, wherein the peak positions and relative intensities of the diffraction peaks are as shown in Table 2 below:

**Table 2. XRPD pattern analysis data of the near-amorphous form of the compound of formula (I)**

| **No.** | **2θ angle (°)** | ***d-*Spacing (Å)** | **Relative intensity (%)** | **Peak height** | **No.** | **2θ angle (°)** | ***d-*Spacing (Å)** | **Relative intensity (%)** | **Peak height** |
|---|---|---|---|---|---|---|---|---|---|
| 1 | 4.341 | 20.3387 | 100.0 | 85 | 4 | 18.955 | 4.6780 | 60.0 | 51 |
| 2 | 5.082 | 17.3732 | 64.7 | 55 | 5 | 23.319 | 3.8115 | 61.2 | 52 |
| 3 | 6.921 | 12.7614 | 75.3 | 64 | 6 | 26.108 | 3.4103 | 58.8 | 50 |

The present disclosure further provides a use of the compound of formula (I) and the crystal form thereof in the manufacture of a medicament for treating psoriasis and/or inflammatory bowel disease.

The present disclosure further provides a use of the Form 1 crystal form of the compound of formula (I) in the manufacture of a medicament for treating psoriasis and/or inflammatory bowel disease.

The present disclosure further provides a use of the near-amorphous form of the compound of formula (I) in the manufacture of a medicament for treating psoriasis and/or inflammatory bowel disease.

### Technical effects

The process for synthesizing the compound of formula (I) and the method for preparing the crystal form thereof provided by the present disclosure offer a novel method for synthesizing pyrazolopyrazine compounds. The beneficial effects include: avoiding the consecutive use of precious metal catalysts and boronic acid reagents over multiple steps, being environmentally friendly, and cost-saving; selective alkylation of pyrazole compounds significantly improves reaction yields; chiral resolution using chiral reagents eliminates the need for expensive chiral SFC separation, and the synthesized final product exhibits extremely high chiral purity; each reaction step is easy to purify, reducing the need for column chromatography purification throughout the process, lowering reaction costs, shortening the cycle, and enhancing efficiency. The crystal form of the compound of formula (I) exhibits excellent chemical stability.

In the *in vitro* activity tests against the four kinase subtypes JAK1, JAK2, JAK3, and TYK2, the compound of formula (I) exhibits good selective inhibition against TYK2 and/or JAK1 and/or JAK2. In mice, the compound of formula (I) has good oral bioavailability and high exposure, which is conducive to producing good *in vivo* efficacy. The compound of the present disclosure exhibits no significant effect on the body weight of animals in the IL-23-induced epidermal hyperplasia model of the C57BL/6 mouse ear pinna, inhibits the increase in thickness on the modeled ear to varying degrees, and shows a good dose-response relationship in reducing the weight of the animal's modeled ear. Oral administration of the compound of formula (I) can significantly improve the degree of paw swelling, ameliorate the scores and relative scores in mice, while having no significant effect on animal body weight.

### Definition and description

Unless otherwise specified, the following terms and phrases, when used herein, have the following meanings. A specific phrase or term should not be considered indefinite or unclear in the absence of a particular definition, but should be understood in the ordinary sense. When a trading name appears herein, it is intended to refer to its corresponding commodity or active ingredient thereof.

Unless otherwise specified, the absolute configuration of a stereogenic center is represented by a wedged solid bond ( 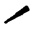 ) and a wedged dashed bond ( 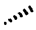 ), and the relative configuration of a stereogenic center is represented by a straight solid bond ( 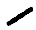 ) and a straight dashed bond ( 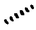 ), a wave line ( 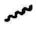 ) is used to represent a wedged solid bond ( 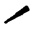 ) or a wedged dashed bond ( 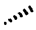 ), or the wave line ( 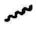 ) is used to represent a straight solid bond ( 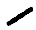 ) or a straight dashed bond ( 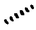 ).

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value or ee%) is 80%.

Optically active (*R*)- and (*S*)-isomers, or *D* and *L* isomers can be prepared using chiral synthesis, chiral reagents, or other conventional techniques. If one kind of enantiomer of a certain compound of the present disclosure is to be obtained, it can be obtained by asymmetric synthesis or derivative action of a chiral auxiliary, wherein the resulting diastereomeric mixture is separated and the auxiliary group is cleaved to provide the pure desired enantiomer. Alternatively, when the molecule contains a basic functional group (such as amino) or an acidic functional group (such as carboxyl), a salt of a diastereoisomer is formed with an appropriate optically active acid or base, and then diastereomeric resolution is performed by conventional methods known in the art, and then the pure enantiomer is recovered. In addition, the enantiomer and the diastereoisomer are generally separated through chromatography, which uses a chiral stationary phase and optionally combines with a chemical derivative method (such as carbamate generated from amine).

The intermediate compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include, but are not limited to, the examples of the present disclosure. Those skilled in the art may appropriately modify aspects such as raw materials and process conditions based on the content of the present disclosure to achieve corresponding other objectives. Such related modifications do not depart from the content of the present disclosure. All similar substitutions and alterations are obvious to those skilled in the art and are deemed to be included within the scope of the present disclosure.

The chemical reactions of the specific embodiments of the present disclosure are completed in a suitable solvent, and the solvent must be appropriate for the chemical changes of the present disclosure and the required reagents and materials thereof. In order to obtain the compounds of the present disclosure, it is sometimes necessary for those skilled in the art to modify or select the synthetic steps or reaction processes on the basis of the existing embodiments.

An important consideration in any synthetic route planning by those skilled in the art is the selection of appropriate protecting groups for reactive functional groups (such as the amino group in the present disclosure).

For any given crystal form, the relative intensities of diffraction peaks may vary due to factors such as preferred orientation caused by crystal morphology, which is well-known in the field of crystallography. Where preferred orientation effects exist, the peak intensities are altered, but the diffraction peak positions of the crystal form remain unchanged. Furthermore, for any given crystal form, slight deviations in peak positions may exist, which is also well-known in the field of crystallography. For example, due to temperature fluctuations during sample analysis, sample movement, or instrument calibration, peak positions may shift, with measurement errors for 20 values sometimes around ± 0.200°. Therefore, it is well-known to those skilled in the art that such variations should be taken into account when determining each crystal structure.

DSC measures the transition temperature at which a crystal absorbs or releases heat due to changes in its crystal structure or the melting of the crystal. For the same crystal form of the same compound, in consecutive analyses, the thermal transition temperature and melting point typically vary within approximately 5°C or 3°C. When we state that a compound exhibits a given DSC peak or melting point, it refers to the DSC peak or melting point ± 5°C or ± 3°C. DSC provides an auxiliary method for distinguishing different crystal forms. Different crystal forms can be identified based on their distinct transition temperature characteristics. It should be noted that for mixtures, their DSC peaks or melting points may vary over a broader range. Furthermore, since decomposition accompanies the melting process of the substance, the melting temperature is related to the heating rate.

For the same crystal form, the TGA weight loss temperature may vary due to factors such as the measuring instrument, method/conditions, *etc.* For any specific crystal form, the weight loss temperature may have an error, which can be approximately ± 5°C or approximately ± 3°C.

It should be noted that during the manufacture of drug crystal forms, it is difficult to avoid the formation of cocrystals between drug molecules and solvent molecules due to external conditions and internal factors, resulting in residual solvent molecules in the solid material, thereby forming solvates, including stoichiometric solvates and non-stoichiometric solvates. The solvates are all included within the scope of the present disclosure.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuKα radiation as the light source and scanning mode: φ/ω scan, and after collecting the relevant data, the crystal structure is further analyzed by direct method (Shelxs97), so that the absolute configuration can be confirmed.

Unless otherwise specified, in the DSC pattern, the upward peak is exothermic.

All solvents used in the present disclosure are commercially available, and commercially available compounds are referred to by their supplier catalog names. When adding a mixed solvent to the reaction mixture, the individual solvents may be mixed first and then added to the reaction mixture; or each single solvent may be sequentially added to the reaction mixture and mixed within the reaction system. The present disclosure adopts the following abbreviations: eq represents equivalent; Boc represents *tert*-butoxycarbonyl; EtOAc represents ethyl acetate; HATU represents 2-(7-azabenzotriazol-1-yl)-*N,N,N',N*'-tetramethyluronium hexafluorophosphate; DBU represents 1,8-diazabicyclo[5.4.0]undec-7-ene; ACN represents acetonitrile; DIEA represents *N,N*-diisopropylethylamine; Im represents imidazole; DCM represents dichloromethane; DMF represents *N,N*-dimethylformamide; PPh₃ represents triphenylphosphine; M represents mol/L or mole per liter; SFC represents supercritical fluid chromatography.

Compounds are named by conventional nomenclature methods in the art or using ChemDraw^{®} software, and commercially available compounds adopt the catalog names provided by suppliers.

The present disclosure is described in detail by the examples below, but these examples do not imply any restrictions on the present disclosure.

### BRIEF DESCRIPTION OF THE DRAWINGS

FIG. 1: XRPD pattern of the Form 1 crystal form of the compound of formula (I);
FIG. 2: DSC pattern of the Form 1 crystal form of the compound of formula (I);
FIG. 3: TGA pattern of the Form 1 crystal form of the compound of formula (I);
FIG. 4: XRPD pattern of the amorphous form of the compound of formula (I);
FIG. 5: DSC pattern of the amorphous form of the compound of formula (I);
FIG. 6: TGA pattern of the amorphous form of the compound of formula (I).

### Instruments and analytical methods

**Table 3. Instrument information and detection method parameters**

| X-ray powder diffractometer (XRPD) and hot-stage XRPD | | | |
|---|---|---|---|
| Instruments | Model | Bruker D8 Advance Diffractometer | |
| | No. | LY-01-034 | |
| | Technical indicators | Copper target wavelength of 1.54Å Kα radiation (40 KV, 40 mA), θ-2θ goniometer, nickel filter, Lynxeye detector | |
| | Acquisition software | Diffrac Plus XRD Commander | |
| | Calibration substance | Corundum (Al₂O₃) | |
| | Analysis software | MDI Jade | |
| Attachment | Non-reflective sample plate | Specification | 24.6 mm diameter ×1.0 mm thickness |
| | | Manufacturer | MTI corporation |
| | Variable temperature stage | Manufacturer | Shanghai Weitu Instrument Technology Development Co., Ltd. |
| | | Sample plate material | Copper plate |
| Parameters | Detection angle | 3°-40° 2θ | |
| | Step size | 0.02° 2θ | |
| | Speed | 0.2 s.step⁻¹ | |
| | Sample detection quantity | >2 mg | |
| | Note | Unless otherwise specified, the samples are not ground before testing | |
| | | | |

| Differential scanning calorimeter (DSC) | | | |
|---|---|---|---|
| Instruments | Model | TA Instruments Q200 DSC | |
| | No. | LY-01-002 | |
| | Control software | Thermal Advantage | |
| | Analysis software | Universal Analysis | |
| | Sample tray | Aluminum crucible (covered without perforation/covered with perforation) | |
| Parameters | Sample detection quantity | 0.5 mg-5 mg | |
| | Protective gas | Nitrogen | |
| | Gas flow rate | 50 mL/min | |
| | Detection method | Equilibrate at 25°C; | |
| | | Ramp 10°C/min to 250°C | |
| | | | |

| Thermogravimetric analyzer (TGA) | | | |
|---|---|---|---|
| Instruments | Model | TA Instruments Q500 TGA | |
| | No. | LY-01-003 | |
| | Control software | Thermal Advantage | |
| | Analysis software | Universal Analysis | |
| | Sample tray | Platinum crucible | |
| Parameters | Sample quantity | 1 mg-10 mg | |
| | Protective gas | Nitrogen | |
| | Gas flow rate | 40 mL/min | |
| | Detection method | Hi-Res sensitivity 3.0; | |
| | | Ramp 10.00°C/min, res 5.0 to 150.00°C; | |
| | | Ramp 10.00°C/min to 400°C | |

### DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed; for those skilled in the art, it is obvious to make various modifications and improvements to the specific examples of the present disclosure without departing from the spirit and scope of the present disclosure.

### Example 1: Preparation of the compound of formula (I)

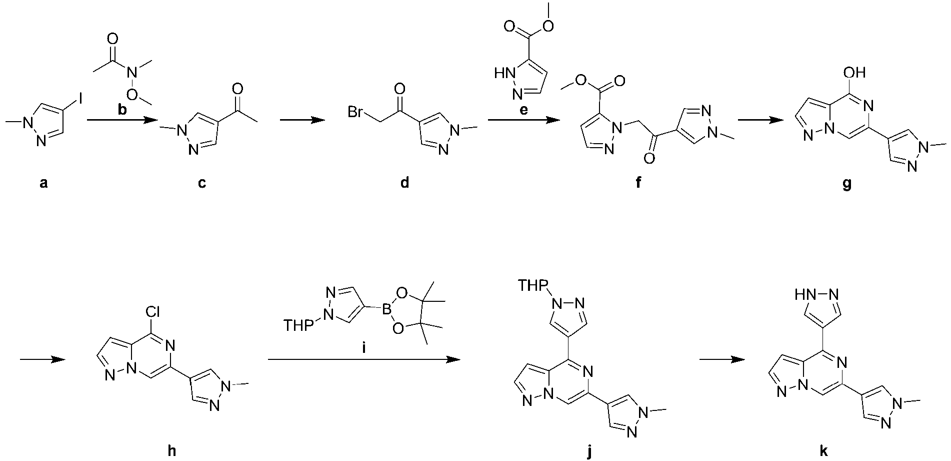

### Step 1:

Compound a (2.5 kg, 12.02 mol, 1 eq) was dissolved in tetrahydrofuran (25 L) (clear), and isopropylmagnesium chloride tetrahydrofuran solution (2 M, 6.31 L, 1.05 eq) was slowly added dropwise under nitrogen flow at 5°C (during the addition of the Grignard reagent, the system became turbid, and the temperature increased, controlled not to exceed 10°C). After the dropwise addition was complete, the reaction mixture was stirred at the same temperature for 30 minutes. Compound b (1.24 kg, 12.02 mol, 1.28 L, 1 eq) was dissolved in tetrahydrofuran (2.5 L) and added at 5°C. After the addition was complete, the internal temperature was raised to 30°C, and the reaction mixture was stirred for 4 hours until the reaction became clear. The reaction system was cooled to 5-10°C. Concentrated hydrochloric acid (12 M, 1.20 L, 1.2 eq) was diluted with water (10 L) and added to the reaction mixture to quench the reaction. Industrial salt (2 kg) was then added to saturate the aqueous phase, and the mixture was allowed to stand for phase separation. The organic phase was separated, and the aqueous phase was extracted three times with a mixed solvent of tetrahydrofuran/ethyl acetate (1:1, 6 L). The organic phases were combined and concentrated. A total of 6 kg (crude product) of compound c was obtained from 4 batches of the above parallel reactions as a yellow oily liquid, which solidified after standing for a period of time. The crude product was directly used in the next step. Step 2:

Compound c (1500 g, 12.08 mol, 1 eq) was dissolved in dichloromethane (24 L) and ethanol (6 L), and pyridinium tribromide (3.67 kg, 11.48 mol, 0.95 eq) was added at 10-15°C. The reaction mixture was stirred at an internal temperature of 25°C for 2 hours. TLC (petroleum ether: ethyl acetate = 1:1) showed that the reaction was complete. Water (5 L) was added to the reaction mixture to quench the reaction, followed by phase separation. The aqueous phase was further extracted with dichloromethane (3 L × 3). The organic phases were combined and concentrated to obtain a crude product. Ethanol/petroleum ether (1:3, 3 times the volume) was added to the crude product. The mixture was slurried and filtered. The filter cake was washed with ethanol/petroleum ether (1:3) and dried in a vacuum oven at 50°C. A total of 7 kg (crude product) of compound d was obtained from 4 batches of the above parallel reactions as a light yellow solid. ¹H NMR (400 MHz, CDCl₃) δ 7.99 (s, 1H), 7.97 (s, 1H), 4.19 (s, 2H), 3.97 (s, 3H).

### Step 3:

Compound e (1.09 kg, 8.62 mol, 1 eq) and compound d (1750 g, 8.62 mol, 1 eq) were dissolved in tetrahydrofuran (20 L). After complete dissolution, magnesium bromide (334.08 g, 1.72 mol, 95% purity, 0.20 eq) was added, followed by slow dropwise addition of *N,N-*diisopropylethylamine (1.34 kg, 10.34 mol, 1.80 L, 1.2 eq) at 15 to 20 °C. The dropwise addition was completed over 3 hours, and the reaction was continued at the same temperature for an additional 15 hours. Water (5 L) and ethyl acetate (10 L) were added to the reaction mixture, and the phases were separated. The aqueous phase was further extracted with dichloromethane (5 L × 2), and the combined organic phases were concentrated. A total of 10 kg (crude product) of compound f was obtained from 4 batches of the above parallel reactions as a pale yellow solid. LCMS (ESI): m/z: 249.1 [M+1]⁺.

### Step 4:

Compound f (1.25 kg, 5.04 mol, 1 eq) and ammonium acetate (1500 g, 19.46 mol, 3.86 eq) were dissolved in ethanol (5 L), and the reaction mixture was stirred in a high-pressure reactor at 120°C for 16 hours. The reaction mixture was taken out from the high-pressure reactor, the solvent was concentrated under vacuum, water (5 L) was added for slurrying, and the mixture was filtered. The filter cake was washed with water. The filter cakes from 8 batches of the above parallel reactions were combined and dried in a vacuum oven (80°C) to obtain compound g (3.9 kg, yield: 44.98 %) as an off-white solid. ¹H NMR (400 MHz, DMSO-d₆) δ 11.45 (br s, 1H), 8.28 (s, 1H), 8.10 (s, 1H), 8.04 (s, 1H), 7.88 (s, 1H), 6.99 (s, 1H), 3.87 (s, 3H).

### Step 5:

Compound g (1300 g, 6.04 mol, 1 eq) was dissolved in acetonitrile (26 L), followed by the addition of phosphorus oxychloride (5.56 kg, 36.24 mol, 3.38 L, 6 eq). The reaction mixture was stirred at 90 °C for 12 hours. The reaction mixture was concentrated to obtain a crude product. Water (40 L) was added to the crude product, and the mixture was stirred for 30 minutes, then filtered. The filter cake was washed with water (5 L × 5) until the aqueous phase reached a pH of 6-7. The filter cake was dried in a vacuum oven at 80°C. A total of 3.7 kg (yield: 87.38%) of compound h was obtained from 3 batches of the above parallel reactions as an off-white solid. LCMS (ESI): m/z: 234.0 [M+1]⁺.

### Step 6:

Compound h (1850 g, 7.92 mol, 1 eq), compound i (2.31 kg, 8.31 mol, 1.05 eq), [1,1'-bis(diphenylphosphino)ferrocene]dichloropalladium(II) (173.80 g, 237.53 mmol, 0.03 eq), and sodium carbonate (2.10 kg, 19.79 mol, 2.5 eq) were dissolved in dioxane (20 L) and water (4 L). The system was purged with nitrogen three times, and the reaction mixture was stirred at 80°C under a nitrogen atmosphere for 12 hours. The reaction mixture was cooled to 20°C, and water (15 L) and ethyl acetate (10 L) were added, followed by stirring and filtration. The mother liquor was allowed to stand for phase separation, and the aqueous phase was extracted once with ethyl acetate (5 L). The organic phases were combined, and mercapto silica gel (1 kg) was added, followed by stirring at 50°C for 12 hours. The mixture was directly filtered, and the filtrate was concentrated. A total of 5.53 kg (crude product) of compound j was obtained from 2 batches of the above parallel reactions as a yellow solid. LCMS (ESI): m/z: 350.2 [M+1]⁺.

### Step 7:

Compound j (3 kg, 8.59 mol, 1 eq) was dissolved in dichloromethane (30 L), followed by the addition of hydrogen chloride/ethyl acetate (4 M, 4.13 L, 2.10 eq). The reaction mixture was stirred at 20°C for 12 hours. The reaction mixture was directly filtered, and the filtrate was discarded. The filter cake was washed with dichloromethane (5 L) to obtain a pale yellow solid. The solid was returned to a 50 L reactor, and water (30 L) was added. An aqueous sodium carbonate solution (10 L, sodium carbonate (1.00 kg, 9.45 mol, 1.2 eq)) was added in portions until the pH reached 9-10. The mixture was slurried at 20°C for 12 hours. The mixture was filtered. The filter cake was washed with water and dried in a vacuum oven (80°C). A total of 3.9 kg (yield: 93.39%) of compound k was obtained from 2 batches of the above parallel reactions as a yellow solid. LCMS (ESI): m/z: 266.1 [M+1]⁺.

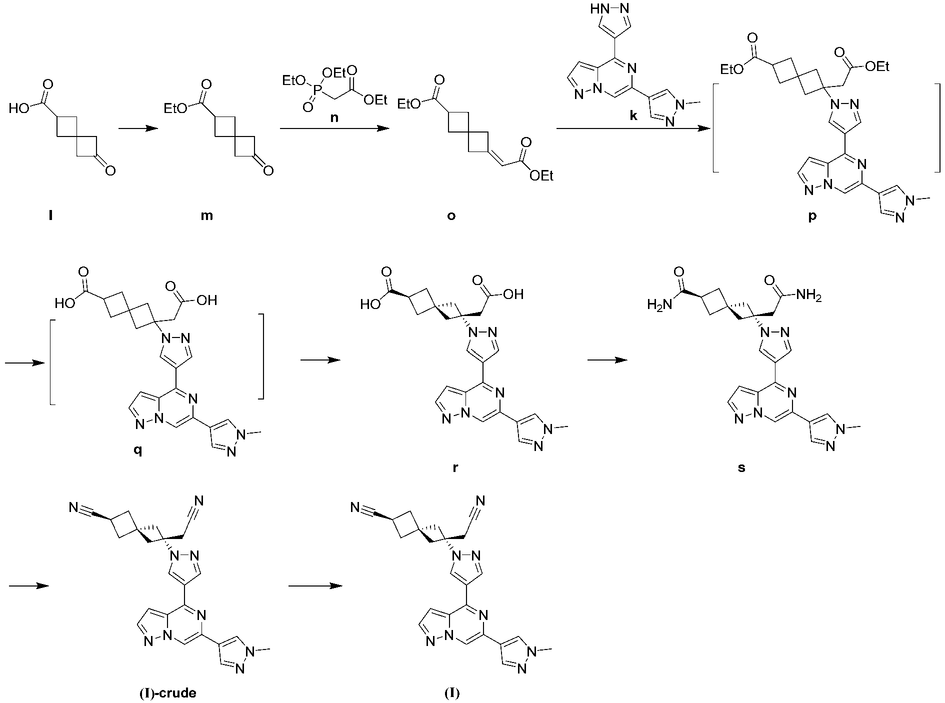

### Step 8:

Compound 1 (3 kg, 19.46 mol, 1 eq) was dissolved in ethanol (18 L), and sulfuric acid (190.86 g, 1.95 mol, 103.73 mL, 0.1 eq) was added at 20°C. The reaction mixture was then stirred at 90°C for 2 hours. Water (15 L) and ethyl acetate (10 L) were added to the reaction mixture under an ice bath, followed by stirring and extraction with ethyl acetate (10 L × 2). The organic phases were combined, washed with an aqueous sodium chloride solution (10 L), dried over anhydrous sodium sulfate, and rotary evaporated to dryness to obtain compound m (6.9 kg, yield: 97.30%) as a brown liquid. ¹H NMR (400MHz, CDCl₃) δ 4.07 - 4.00 (m, 2H), 3.07 - 2.99 (m, 3H), 2.98 - 2.95 (m, 2H), 2.48 - 2.39 (m, 2H), 2.38 - 2.29 (m, 2H), 1.17 - 1.14 (m, 3H).

### Step 9:

Compound n (4.52 kg, 20.17 mol, 4.00 L, 1.05 eq) was dissolved in tetrahydrofuran (30 L), and compound m (3.5 kg, 19.21 mol, 1 eq), lithium bromide (2.50 kg, 28.81 mol, 1.5 eq), and triethylamine (2.92 kg, 28.81 mol, 4.01 L, 1.5 eq) were added thereto at 0°C. The reaction mixture was then stirred at 25°C for 12 hours. The reaction mixture was quenched with water (10 L) and extracted with ethyl acetate (10 L × 3). The organic phases were combined, washed with an aqueous sodium chloride solution (10 L), dried over anhydrous sodium sulfate, filtered, and concentrated to obtain a crude product. The crude product was dissolved in petroleum ether/ethyl acetate (10:1, 12 L) and purified by column chromatography (petroleum ether/ethyl acetate = 10:1) to obtain compound o (9.3 kg, yield: 92.50%) as a yellow liquid. LCMS (ESI): m/z: 253.0 [M+1]⁺. ¹H NMR (400MHz, CDCl₃) δ 5.50 (br s, 1H), 4.13 - 3.92 (m, 4H), 3.07 (br s, 1H), 2.98 (br s, 1H), 2.95 - 2.84 (m, 1H), 2.77 (br s, 1H), 2.70 (br s, 1H), 2.32 - 2.12 (m, 4H), 1.20 - 1.08 (m, 6H).

### Step 10:

At 20 to 30°C, acetonitrile (20 L), compound k (1.0 kg, 3.48 mol, 1 eq), 1,8-diazabicyclo[5.4.0]undec-7-ene (555 g, 3.66 mol, 1.05 eq), and compound o (945 g, 3.48 mol, 1 eq) were successively added to a 50 L reactor. The reaction mixture was heated to 85 to 95°C (internal temperature 82°C) and stirred for 14 hours to obtain compound p. The reactor was cooled to 20 to 30°C, and an aqueous sodium hydroxide solution (20 L; 417.6 g of sodium hydroxide dissolved in 20 L of water) was added and stirred for 2 hours. Concentrated hydrochloric acid (1.25 L) was added to the reactor at 20 to 30°C to adjust the pH to 4 to 5, and the mixture was stirred at this temperature for 12 hours. The mixture was filtered, and the filter cake was washed with water (5 L). The filter cakes obtained from 2 batches of the above parallel reactions were vacuum-dried at 80°C for 72 hours to obtain compound q (2.7 kg, yield: 74.94%) as a light yellow powder. LCMS (ESI): m/z: 462.2 [M+1]⁺.

At 20 to 30°C, tetrahydrofuran (25 L), water (1.5 L), compound q (1.7 kg, 3.37 mol, 1 eq), and (R)-(+)-N-benzyl-1-phenylethylamine (1.5 kg, 7.08 mol, 2.1 eq) were sequentially added to a 50 L reactor. The reaction mixture was stirred at 20 to 30°C for 1 hour, heated to 73°C and stirred for 2 hours, then naturally cooled to 20 to 30°C and stirred at this temperature for 12 hours. The reaction mixture was filtered, and the filter cake was washed three times with tetrahydrofuran/water (16.5:1; 10 L). The filtrate was concentrated under reduced pressure, slurried with an aqueous sodium carbonate solution (15 L; 855 g of sodium carbonate dissolved in 15 L of water) for 1 hour, and ethyl acetate (10 L) was added, followed by stirring for another 1 hour. The mixture was allowed to stand for phase separation, and the aqueous phase was washed with ethyl acetate (10 L). The organic phases from both washes were discarded. The aqueous phase was transferred to a reactor, and acetonitrile (1.5 L, water: acetonitrile = 10:1 in the reactor) was added. The pH was adjusted to 4 to 5 with concentrated hydrochloric acid (1.3 L), and the mixture was stirred at 20 to 30°C for 12 hours, during which a white solid formed. The reaction mixture was filtered, and the filter cakes obtained from 2 batches of the above parallel reactions were vacuum-dried at 70°C for 12 hours to obtain compound r (1.4 kg) as an off-white powder. At 20 to 30°C, tetrahydrofuran (33 L), water (2 L), the above compound r (1.4 kg, 3.03 mol, 1 eq), and (*S*)-(-)-*N*-benzyl-1-phenylethylamine (1.35 kg, 6.37 mol, 2.1 eq) were sequentially added to a 50 L reactor. The reaction mixture was stirred at 20 to 30°C for 1 hour, then heated to 73°C and stirred for 2 hours. The reaction mixture was naturally cooled to 20 to 30°C and stirred at this temperature for 12 hours. The reaction mixture was filtered, and the filter cake was washed three times with tetrahydrofuran/water (16.5:1; 10 L). At 20 to 30°C, tetrahydrofuran (33 L), water (2 L), and the above filter cake were sequentially added to a 50 L reactor, stirred at 20 to 30°C for 1 hour, heated to 73°C and stirred for 2 hours, naturally cooled to 20 to 30°C, and stirred at this temperature for 12 hours. The reaction mixture was filtered, and the filter cake was washed three times with tetrahydrofuran/water (16.5:1; 20 L). The filter cake was slurried with an aqueous sodium carbonate solution (20 L; 771.68 g of sodium carbonate dissolved in 20 L of water) for 1 hour, and ethyl acetate (10 L) was added. The mixture was stirred for another 1 hour and allowed to stand for phase separation. The aqueous phase was washed with ethyl acetate (20 L). The organic phases from both washes were discarded. The aqueous phase was transferred to a reactor, acetonitrile (2 L; water: acetonitrile = 10:1 in the reactor) was added, and the pH was adjusted to 4 to 5 with concentrated hydrochloric acid (1.18 L). The mixture was stirred at 20 to 30°C for 12 hours, during which a white solid formed. The reaction mixture was filtered, and the filter cake was vacuum-dried at 70°C for 12 hours to obtain compound r (1.08 kg, yield: 31.75%) as an off-white powder. SFC analysis method (column type: Chiralcel OJ-3 100 × 4.6 mm I.D., 3 µm; mobile phase: A: CO₂; B: methanol (0.05% diethylamine); B-phase gradient: 5%-40% for 4 min, maintaining 40% for 2.5 min, 5% for 1.5 min; flow rate: 2.8 mL/min); retention time: 3.285 minutes; ee% = 99.02%.

### Step 11:

At 15 to 25°C, *N*,*N*-dimethylformamide (3 L), compound r (1.08 kg, 2.32 mol, 1 eq), *N*,*N*-diisopropylethylamine (1.62 L, 9.29 mol, 4 eq), 2-(7-azabenzotriazol-1-yl)-*N,N,N',N'-*tetramethyluronium hexafluorophosphate (1.86 kg, 4.88 mol, 2.1 eq), and ammonium chloride (261.01 g, 4.88 mol, 2.1 eq) were sequentially added to a 10 L three-necked flask, and the reaction mixture was stirred at 15 to 25°C for 1 hour. The internal temperature of a 50 L reactor was maintained at 20°C. Dichloromethane (20 L) was first transferred into the reactor under reduced pressure, followed by transferring the reaction mixture from the 10 L three-necked flask into the reactor. Finally, additional dichloromethane (10 L) was transferred into the reactor, and the temperature was maintained for slurrying for 16 hours. The mixture was filtered, and the filter cake was washed with dichloromethane (5 L). The filter cake was vacuum-dried at 50°C for 16 hours to obtain compound s (1.055 kg, yield: 82.77%) as an off-white powder. LCMS (ESI): m/z: 460.2 [M+1]⁺.

### Step 12:

Dichloromethane (20 L) was added to a 50 L reactor at 20 to 30°C, and the mixture was cooled to 0°C with stirring. At 0°C, compound s (1.055 kg, 1.94 mol, 1 eq) and triethylamine (1.08 L, 7.75 mol, 4 eq) were sequentially added to the reactor, followed by the slow dropwise addition of trifluoroacetic anhydride (1.08 L, 7.75 mol, 4 eq) at 0°C. The reactor was then warmed to 15 to 25°C and stirred at this temperature for 1 hour. The reaction was quenched with a saturated aqueous sodium carbonate solution (10 L), and dichloromethane (5 L) was added for extraction. The organic phase was concentrated and then slurried with methanol/methyl *tert-*butyl ether (12 L; 5:1) for 12 hours to obtain a white solid. The mixture was filtered, and the filter cake was vacuum-dried at 50°C for 12 hours to obtain compound (I)-crude (670 g, yield: 82.77%) as an off-white powder. LCMS (ESI): m/z: 424.1 [M+1]⁺. ¹H NMR (400MHz, DMSO-d₆) δ 9.02 (s, 1H), 8.79 (s, 1H), 8.46 (s, 1H), 8.36 (s, 1H), 8.19 (d, *J*=2.5 Hz, 1H), 8.15 (s, 1H), 7.43 (dd, *J*=0.8, 2.5 Hz, 1H), 3.91 (s, 3H), 3.36 - 3.34 (m, 2H), 3.33 - 3.24 (m, 2H), 3.03 - 2.93 (m, 2H), 2.68 - 2.58 (m, 3H), 2.41 - 2.30 (m, 2H).

### Step 13:

Methanol (6.7 L) and compound (I)-crude (335 g, 0.79 mol, 1 eq) were successively added to a 10 L three-necked flask at 20 to 30°C, and the reaction mixture was stirred at 90°C for 1 hour until the solution became clear. The reaction mixture was filtered while hot, and the resulting filtrate was stirred at 20 to 30°C for 12 hours, during which a white solid precipitated. The reaction mixtures from 2 batches of the above parallel reactions were combined and filtered, and the filter cake was vacuum-dried at 50°C for 12 hours to obtain filter cake 1 (576 g).

At 20 to 30°C, methanol (5.76 L) and the above filter cake 1 (288 g, 0.68 mol, 1 eq) were successively added to a 10 L three-necked flask, and the reaction mixture was stirred at 90°C for 1 hour until the reaction mixture became clear. The heating was stopped, and the temperature was slowly lowered to 20 to 30°C. The reaction mixture was stirred at this temperature for 12 hours, during which a white solid precipitated. The reaction mixtures from 2 batches of the above parallel reactions were combined and filtered, and the filter cake was vacuum-dried at 50°C for 12 hours to obtain filter cake 2 (439 g).

At 20 to 30°C, methanol (8.78 L), the above filter cake 2 (439 g, 1.04 mol, 1 eq), and triethylamine (72.25 mL, 0.52 mol, 0.5 eq) were successively added to a 10 L three-necked flask. The reaction mixture was stirred at 90°C for 1 hour until the reaction mixture became clear. The heating was stopped, and the temperature was slowly lowered to 20 to 30°C. The reaction mixture was stirred at this temperature for 12 hours, during which a white solid precipitated. The reaction mixture was filtered, and the filter cake was vacuum-dried at 50°C for 12 hours to obtain the compound of formula (I) (370 g, yield: 55.23%) as an off-white powder. LCMS (ESI): m/z: 424.2 [M+1]⁺. The SFC analysis method (column type: Cellulose 2 100 × 4.6 mm I.D., 3 µm; mobile phase: A: CO₂; B: methanol (0.05% diethylamine); B-phase gradient: B: 40%; flow rate: 2.8 mL/min); retention time: 5.586 minutes; ee% = 100%.

### Example 2: Preparation of the near-amorphous form of the compound of formula (I)

The compound of formula (I) (2 g, 4.72 mmol) was purified by preparative HPLC (column type: Phenomenex C18 80 × 40 mm × 3 µm; mobile phase: [water (NH₃H₂O + NH₄HCO₃) - acetonitrile]; acetonitrile%: 36%-66%; 8 min) and then lyophilized to obtain the near-amorphous form of the compound of formula (I) (1.7 g, yield: 83.73%) as a white solid.

Approximately 50 mg of the near-amorphous sample of the compound of formula (I) was taken, added with 1 mL of water, and the slurry was allowed to crystallize at room temperature for 30 minutes. After centrifugation, the solid was vacuum-dried at 40°C overnight, and the resulting solid was subjected to XRPD characterization. The results showed that the solid was nearly amorphous.

Approximately 50 mg of the near-amorphous sample of the compound of formula (I) was taken, placed in a centrifuge tube, and left standing in a bottle containing water at room temperature for 3 days. The resulting solid was subjected to XRPD characterization. The results showed that the solid was nearly amorphous.

### Example 3: Preparation of the Form 1 crystal form of the compound of formula (I)

The compound of formula (I) obtained in Example 1 was the Form 1 crystal form. Other preparation methods for the Form 1 crystal form are shown in Table 4:

**Table 4. Crystal form preparation methods**

| Preparation method | State | Preparation method |
|---|---|---|
| Evaporative crystallization | Solution | The sample solution was evaporated to dryness |
| Crystal slurry crystallization | Suspension | The sample suspension was stirred for a period of time |
| Cooling crystallization | Solution | The sample solution was cooled to precipitate powder |
| Anti-solvent crystallization | Solution | An anti-solvent was added to the sample solution (or the solution was added to the anti-solvent), and a powder was precipitated |
| Gas-solid diffusion crystallization | Powder | The sample powder was exposed to another solvent atmosphere for a period of time |
| Term explanation | | |
| "Room temperature" or "RT": 10°C-30°C, >30% RH; | | |
| "Solvent ratio": solvent volume ratio; | | |
| "Overnight": spanning nighttime; | | |
| "Solution": a system with no visible solids to the naked eye under no additional light source; | | |

### 1. Evaporative crystallization method

Approximately 50 mg of a near-amorphous sample of the compound of formula (I) was taken, respectively, and the corresponding solvent (Table 5) was added. After the sample was completely dissolved and filtered, the filtrate was placed at the corresponding temperature (Table 5) and allowed to evaporate to dryness through small holes. The resulting solid was subjected to XRPD characterization. The results are shown in Table 5.

**Table 5. Results of preparing Form 1 crystal form by evaporative crystallization method**

| No. | Temperature (°C) | Solvent 1 | Solvent 2 | Solvent 1/Solvent 2 (mL) | Result analysis |
|---|---|---|---|---|---|
| 1 | RT | Tetrahydrofuran | Chloroform | 0.1/0.1 | Form 1 |
| 2 | 40 | Acetonitrile | Ethyl acetate | 0.1/0.1 | Form 1 |

### 2. Crystal slurry crystallization method

(1) Approximately 50 mg of the Form 1 crystal form sample of the compound of formula (I) was taken, respectively, and after adding the corresponding solvent (Table 6), the slurry was subjected to slurry crystallization at the corresponding temperature (Table 6) for 3 days, followed by centrifugation and vacuum-drying at 40°C overnight. The resulting solid was subjected to XRPD characterization. The results are shown in Table 6.

**Table 6. Results of preparing Form 1 crystal form by crystal slurry crystallization method**

| No. | Temperature (°C) | Solvent 1 | Solvent 2 | Solvent 1/Solvent 2 (mL) | Result analysis |
|---|---|---|---|---|---|
| 1 | RT | Ethanol | - | 1.0 | Form 1 |
| 2 | 40 | Water | - | 1.0 | Form 1 |
| 3 | 4 | Isopropyl acetate | - | 1.0 | Form 1 |
| 4 | 40 | Ethanol | Water | 0.2/1.0 | Form 1 |
| 5 | RT | Acetone | Water | 0.4/0.8 | Form 1 |
| 6 | RT | Tetrahydrofuran | Water | 0.8/3.2 | Form 1 |
| 7 | 40 | Acetonitrile | Water | 0.1/0.9 | Form 1 |
| 8 | RT | Acetone | Methyl *tert-*butyl ether | 0.2/1.0 | Form 1 |

(2) Approximately 50 mg of the near-amorphous sample of the compound of formula (I) was taken, respectively, and after adding the corresponding solvent (see Table 7), the slurry was subjected to slurry crystallization at the corresponding temperature (see Table 7) for 30 minutes, followed by centrifugation and vacuum-drying at 40°C overnight. The resulting solid was subjected to XRPD characterization. The results are shown in Table 7.

**Table 7. Results of preparing Form 1 crystal form by crystal slurry crystallization method**

| No. | Temperature (°C) | Solvent 1 | Solvent 2 | Solvent 1/Solvent 2 (mL) | Result analysis |
|---|---|---|---|---|---|
| 1 | 40 | Methyl *tert-*butyl ether | - | 1.0 | Form 1 |
| 2 | 4 | Isopropanol | Water | 0.2/1.0 | Form 1 |
| 3 | RT | Acetone | Water | 0.1/1.0 | Form 1 |
| 4 | RT | 1,4-Dioxane | Water | 0.1/1.0 | Form 1 |
| 5 | 40 | Butanone | n-Heptane | 0.1/1.0 | Form 1 |
| 6 | RT | Methanol | Methyl *tert-*butyl ether | 0.1/2.0 | Form 1 |
| 7 | RT | Acetonitrile | Isopropyl ether | 0.1/1.0 | Form 1 |
| 8 | 4 | *n*-Butanol | Toluene | 0.2/2.0 | Form 1 |
| 9 | 40 | Ethyl acetate | Methylcyclohexane | 0.1/2.0 | Form 1 |

### 3. Cooling crystallization method

(1) Approximately 50 mg of the Form 1 crystal form sample of the compound of formula (I) was taken, 5 mL of ethanol was added, and the mixture was dissolved completely in a 60°C water bath. The solution was then transferred to an ice-salt bath and stirred for 0.5 hours to precipitate a solid. After centrifugation, the solid was vacuum-dried at 40°C overnight, and the resulting solid was subjected to XRPD characterization. The results showed that the solid was the Form 1 crystal form.
(2) Approximately 50 mg of the near-amorphous sample of the compound of formula (I) was taken, respectively, and the corresponding solvent (see Table 8) was added. The mixture was dissolved completely in a 60°C water bath for 5 minutes, and then a solid precipitated. The mixture was stirred for 0.5 hours and centrifuged. The resulting solid was subjected to XRPD characterization. The results are shown in Table 8.

**Table 8. Results of preparing Form 1 crystal form by cooling crystallization method**

| Experiment No. | Start-end temperature (°C) | Solvent 1 | Solvent 2 | Solvent volume (mL) | Result |
|---|---|---|---|---|---|
| 1 | 60 | Ethyl acetate | *n*-Heptane | 0.3/0.8 | Form 1 |
| 2 | 60 | Acetone | Water | 0.2/0.8 | Form 1 |
| 3 | 60 | Acetonitrile | Methyl *tert-*butyl ether | 0.2/0.8 | Form 1 |

### 4. Anti-solvent crystallization method

Approximately 50 mg of the Form 1 crystal form sample of the compound of formula (I) was taken, dissolved in solvent 1 from Table 9 until completely dissolved, and the sample solution was then added dropwise to solvent 2 under stirring at room temperature. The mixture was stirred until a solid precipitated and then stirred for another 2 hours. The mixture was centrifuged, and the solid was vacuum-dried at 40°C overnight. The resulting solid was subjected to XRPD characterization. The results are shown in Table 9.

**Table 9. Results of preparing Form 1 crystal form by anti-solvent crystallization method**

| No. | Method | Solvent 1 | Solvent 2 | Solvent 1/Solvent 2 (mL) | Result analysis |
|---|---|---|---|---|---|
| 1 | Reverse addition | Acetone | *n*-Heptane | 0.5/1.0 | Form 1 |
| 2 | Reverse addition | Tetrahydrofuran | *n*-Heptane | 0.5/2.0 | Form 1 |
| 3 | Reverse addition | Dimethyl sulfoxide | Water | 0.1/3.0 | Form 1 |

### 5. Gas-solid diffusion crystallization method

Approximately 50 mg of a near-amorphous sample of the compound of formula (I) was placed in a centrifuge tube and allowed to stand at room temperature in a bottle containing acetone for 3 days. The resulting solid was subjected to XRPD characterization, and the results showed that the solid was the Form 1 crystal form.

### Example 4: Stability test of the Form 1 crystal form of the compound of formula (I)

According to the "Guidelines for Stability Testing of Active Pharmaceutical Ingredients and Preparations" (General Rule 9001 of the Chinese Pharmacopoeia 2020 Edition), in order to evaluate the solid stability of the Form 1 crystal form of the compound of formula (I), influencing factor tests (high temperature, high humidity, and light exposure) and accelerated tests (40°C/75% RH and 60°C/75% RH) were conducted for the stability investigation of the Form 1 crystal form of the compound of formula (I). The test results are shown in Table 10 and Table 11 below.

**Table 10. Crystal form stability results: influencing factors test**

| | 60°C | 25°C/92.5% RH | Light exposure (open container) | Light exposure (control) |
|---|---|---|---|---|
| 5 days | Form 1 | Form 1 | - | - |
| 10 days | Form 1 | Form 1 | Form 1 | Form 1 |

**Table 11. Crystal form stability results: accelerated test**

| Stability conditions | -- | 40°C/75% RH | 60°C/75% RH | | | | | |
|---|---|---|---|---|---|---|---|---|
| Time | 0 month | 10 days | 1 month | 2 months | 3 months | 10 days | 1 month | 2 months |
| Crystal form | Form 1 | Form 1 | Form 1 | Form 1 | Form 1 | Form 1 | Form 1 | Form 1 |

Conclusion: The Form 1 crystal form of the compound of formula (I) exhibits no significant changes in crystal form under all stability conditions, demonstrating good chemical stability.

### Biological activity test

### Experimental example 1: In vitro activity test of JAK1, JAK2, JAK3, and Tyk2 kinases

### Experimental materials

Recombinant human JAK1, JAK2, JAK3, and Tyk2 proteases, along with major instruments and reagents, were provided by Eurofins in the UK.

### Experimental method

JAK2, JAK3, and TYK2 were diluted with: 20 mM 3-(N-morpholino)propanesulfonic acid (MOPS), 1 mM EDTA, 0.01% Brij-35.5% glycerol, 0.1% β-mercaptoethanol, 1 mg/mL BSA; JAK1 was diluted with: 20 mM TRIS, 0.2 mM EDTA, 0.1% β-mercaptoethanol, 0.01% Brij-35.5% glycerol. The compound of formula (I) was prepared as a 100% DMSO solution and diluted to 50 times the final assay concentration. The test compound was subjected to a 3-fold serial dilution, with final concentrations ranging from 10 µM to 0.001 µM across 9 concentrations, and the DMSO content in the detection reaction was 2%. The working stock solution of this compound was added as the first component of the reaction to the assay wells, followed by the addition of the remaining components according to the protocol detailed below.

### JAK1(h) enzyme reaction

JAK1(h) was incubated with 20 mM Tris/HCl pH 7.5, 0.2 mM EDTA, 500 µM MGEEPLYWSFPAKKK, 10 mM magnesium acetate, and [γ-³³P]-ATP (activity and concentration adjusted as needed). The reaction was initiated by adding the Mg/ATP mixture and incubated at room temperature for 40 minutes, then terminated by adding 0.5% phosphoric acid. Then, 10 µL of the reaction mixture was spotted onto a P30 filter pad, washed three times with 0.425% phosphoric acid and once with methanol within 4 minutes, dried, and subjected to scintillation counting.

### JAK2(h) enzyme reaction

JAK2(h) was incubated with 8 mM MOPS pH 7.0, 0.2 mM EDTA, 100 µM KTFCGTPEYLAPEVRREPRILSEEEQEMFRDFDYIADWC, 10 mM magnesium acetate, and [γ-³³P]-ATP (prepared to the required activity and concentration). The reaction was initiated by adding the Mg/ATP mixture and incubated at room temperature for 40 minutes, then terminated by adding 0.5% phosphoric acid. Then, 10 µL of the reaction mixture was spotted onto a P30 filter pad, washed three times with 0.425% phosphoric acid and once with methanol within 4 minutes, dried, and subjected to scintillation counting.

### JAK3(h) enzyme reaction

JAK3(h) was incubated with 8 mM MOPS pH 7.0, 0.2 mM EDTA, 500 µM GGEEEEYFELVKKKK, 10 mM magnesium acetate, and [γ-³³P]-ATP (activity and concentration were adjusted as needed). The reaction was initiated by adding the Mg/ATP mixture and incubated at room temperature for 40 minutes, then terminated by adding 0.5% phosphoric acid. Then, 10 µL of the reaction mixture was spotted onto a P30 filter pad, washed three times with 0.425% phosphoric acid and once with methanol within 4 minutes, dried, and subjected to scintillation counting.

### TYK2(h) enzyme reaction

TYK2(h) was incubated with 8 mM MOPS pH 7.0, 0.2 mM EDTA, 250 µM GGMEDIYFEFMGGKKK, 10 mM magnesium acetate, and [γ-³³P]-ATP (activity and concentration adjusted as needed). The reaction was initiated by adding the Mg/ATP mixture and incubated at room temperature for 40 minutes, then terminated by adding 0.5% phosphoric acid. Then, 10 µL of the reaction mixture was spotted onto a P30 filter pad, washed three times with 0.425% phosphoric acid and once with methanol within 4 minutes, dried, and subjected to scintillation counting.

### Data analysis

The IC₅₀ results were analyzed using XLFIT5 (205 formula) from IDBS, as detailed in Table 12.

**Table 12. In vitro screening test results of the compound of the present disclosure**

| Compound No. | **JAK1** (IC₅₀, nM) | **JAK2** (IC₅₀, nM) | **JAK3** (IC₅₀, nM) | **TYK2** (IC₅₀, nM) |
|---|---|---|---|---|
| Compound of formula (I) | 10 | 4 | 189 | 0.9 |

Conclusion: The compound of formula (I) exhibits good selective inhibition against TYK2 and/or JAK1 and/or JAK2 in *in vitro* activity tests for the four kinase subtypes JAK1, JAK2, JAK3, and TYK2.

### Experimental example 2: Pharmacokinetic (PK) test

The clarified solutions obtained by dissolving the test compound were administered to male mice (balb/c) via tail vein injection and oral gavage, respectively. After administration of the test compound, blood was collected from the mandibular vein at 0.083, 0.25, 0.5, 1, 2, 4, 8, and 24 hours for the intravenous injection group (1 mg/kg) and at 0.25, 0.5, 1, 2, 4, 6, 8, and 24 hours for the oral gavage group (3 mg/kg), followed by centrifugation to obtain plasma. Plasma concentrations were determined by the LC-MS/MS method, and relevant pharmacokinetic parameters were calculated using WinNonlin^{™} Version 6.3 pharmacokinetic software with non-compartmental model linear-log trapezoidal method. The test results are shown in Table 13:

**Table 13. PK test results of the compound of formula (I) in mice**

| PK parameters | Result |
|---|---|
| Cl (mL/kg/min) | 15.6 |
| Vd (L/kg) | 1.59 |
| T_{1/2} (hr) | 2.39 |
| Cₘₐₓ (nM) | 3040 |
| AUC_{0-inf} (nM·hr) | 7673 |
| Bioavailability (%)^{a} | 88.7 |

| | |
|---|---|
| Note: T_{1/2}: half-life; Cₘₐₓ: maximum concentration; Cl: clearance; Vd: volume of distribution; AUC_{0-inf}: area under the plasma concentration-time curve from time 0 extrapolated to infinity; Bioavailability: BA. | |

Conclusion: The compound of formula (I) exhibits good oral bioavailability and high exposure in mice, which is conducive to producing good *in vivo* efficacy.

### Experimental example 3: Pharmacodynamic test of psoriasis model in mice

Objective: The purpose of this experiment is to evaluate the preventive and therapeutic effects of the test substance on IL-23-induced psoriasis-like skin lesions in mice, which were established by intradermal injection of IL-23 into the mouse ear pinna.

Methods: After the acclimation period, 50 mice were divided into 5 groups of 10 each by Excel complete randomization, namely: vehicle (0.5% MC & 0.5% Tween 80) control group, model group, and compound of formula (I) (10, 20, 50 mg/kg) groups. Except for the vehicle control group, the remaining groups were intradermally injected with IL-23 (3 µg/10 µL/mouse/day, QD) in the right ear for 8 consecutive days (D1 to D8), while the control group animals were intradermally injected with an equal volume of normal saline solution (10 µL/mouse/day, QD) in the right ear for 8 consecutive days (D1 to D8). During the modeling period, the vehicle or test drug was orally administered twice daily (Bid) at 6-hour intervals for 8 consecutive days (D1 to D8). During the administration period, the body weight of the animals was monitored every two days. On D1, D3, D5, D7 (all before IL-23 injection), and D9, the thickness of the right ear of the mice was measured, and the appearance of the ear pinna was scored. At the end of the experiment (Day 10), the modeled ear was excised, and an ear piece was punched along the ear pinna edge using an 8 mm punch and weighed. The punched right ear piece was fixed with formalin and subjected to histopathological examination (H&E) staining.

The experimental results are shown in Tables 14-17.

**Table 14. Effects of oral gavage administration of the test substance twice daily for 8 consecutive days on body weight in IL-23-induced epidermal hyperplasia model of the mouse ear pinna**

| Group and dosage | Body weight (g)/day | | | | |
|---|---|---|---|---|---|
| | 1 | 3 | 5 | 7 | 9 |
| Vehicle control group | 19.78 ± 0.60 | 19.88 ± 0.89 | 20.36 ± 0.64 | 20.06 ± 0.78 | 20.23 ± 0.57 |
| Model Group | 19.76 ± 0.56 | 19.76 ± 0.65 | 19.73 ± 0.70 | 19.51 ± 0.87 | 19.90 ± 0.72 |
| Compound of formula (I) 10 mg/kg | 19.77 ± 0.59 | 19.69 ± 0.57 | 20.02 ± 0.77 | 19.69 ± 0.63 | 20.04 ± 0.72 |
| Compound of formula (I) 20 mg/kg | 19.77 ± 0.54 | 19.85 ± 0.78 | 20.13 ± 0.78 | 19.80 ± 0.92 | 20.27 ± 0.90 |
| Compound of formula (I) 50 mg/kg | 19.78 ± 0.53 | 19.85 ± 0.68 | 20.41 ± 0.70 | 20.15 ± 0.56 | 20.28 ± 0.76 |

**Table 15. Effects of oral gavage administration of the test substance twice daily for 8 consecutive days on ear thickness in IL-23-induced epidermal hyperplasia model of the mouse ear pinna**

| Group and dosage | Ear thickness (mm)/day | | | | |
|---|---|---|---|---|---|
| | 1 | 3 | 5 | 7 | 9 |
| Vehicle control group | 0.193 ± 0.005 | 0.218 ± 0.009 | 0.216 ± 0.012 | 0.230 ± 0.017 | 0.227 ± 0.015 |
| Model Group | 0.188 ± 0.007 | 0.303 ± 0.029^{##} | 0.338 ± 0.027^{##} | 0.474 ± 0.068^{##} | 0.520 ± 0.078^{##} |
| Compound of formula (I) 10 mg/kg | 0.189 ± 0.006 | 0.287 ± 0.018 | 0.311 ± 0.013* | 0.373 ± 0.030** | 0.430 ± 0.048* |
| Compound of formula (I) 20 mg/kg | 0.190 ± 0.009 | 0.289 ± 0.028 | 0.306 ± 0.018** | 0.350 ± 0.025** | 0.381 ± 0.032** |
| Compound of formula (I) 50 mg/kg | 0.193 ± 0.006 | 0.279 ± 0.024* | 0.289 ± 0.025** | 0.338 ± 0.023** | 0.352 ± 0.032** |

| | | | | | |
|---|---|---|---|---|---|
| *^{##}P*<0.01 vs. Control **P<0.05, **P<0.01* vs. Model | | | | | |

**Table 16. Effects of test substance on ear weight in IL-23-induced epidermal hyperplasia model of the mouse ear pinna**

| Group and dosage | Ear weight (mg) |
|---|---|
| Vehicle control group | 12.94 ± 0.75 |
| Model Group | 22.80 ± 3.01^{##} |
| Compound of formula (I) 10 mg/kg | 19.16 ± 1.56** |
| Compound of formula (I) 20 mg/kg | 18.76 ± 1.62** |
| Compound of formula (I) 50 mg/kg | 17.63 ± 1.47** |

| | |
|---|---|
| ^{##}*P*<0.01 vs. Control ***P<*0.01 vs. Model | |

**Table 17. Effects of oral gavage administration of the test substance twice daily for 8 consecutive days on the total score of IL-23-induced epidermal hyperplasia in the mouse ear pinna**

| Group and dosage | Ear comprehensive score/day | | | | |
|---|---|---|---|---|---|
| | 1 | 3 | 5 | 7 | 9 |
| Vehicle control group | 0.00 ± 0.00 | 0.00 ± 0.00 | 0.00 ± 0.00 | 0.00 ± 0.00 | 0.00 ± 0.00 |
| Model Group | 0.00 ± 0.00 | 0.20 ± 0.42 | 1.70 ± 0.95## | 5.60 ± 1.43## | 6.40 ± 1.58## |
| Compound of formula (I) 10 mg/kg | 0.00 ± 0.00 | 0.00 ± 0.00 | 0.60 ± 0.52** | 1.70 ± 1.06** | 2.60 ± 1.43** |
| Compound of formula (I) 20 mg/kg | 0.00 ± 0.00 | 0.00 ± 0.00 | 0.60 ± 0.70** | 1.40 ± 0.52** | 1.50 ± 0.71** |
| Compound of formula (I) 50 mg/kg | 0.00 ± 0.00 | 0.00 ± 0.00 | 0.10 ± 0.32** | 1.40 ± 0.70** | 1.00 ± 0.47** |

| | | | | | |
|---|---|---|---|---|---|
| *^{##}P*<0.01 vs. Control ***P<*0.01 vs. Model Note: IL-23: Interleukin-23. | | | | | |

### Conclusion:

(1) In the IL-23-induced epidermal hyperplasia model of the C57BL/6 mouse ear pinna, oral gavage administration of the compound of formula (I) (10, 20, 50 mg/kg) twice daily for 8 consecutive days shows no significant effect on the body weight of the animals in the IL-23-induced epidermal hyperplasia model of the C57BL/6 mouse ear pinna.
(2) Effect on the thickness of the modeled ear in animals: At doses of 10 and 20 mg/kg, compound 1 significantly reduces the thickness of the modeled ear in mice starting from day 5, the compound of formula (I) at a dose of 50 mg/kg significantly reduces the thickness of the modeled ear in animals as early as D3, and all three dose groups exhibit a good dose-response relationship. It is shown that the compound of formula (I) (10, 20, 50 mg/kg) in each group can inhibit the increase in the thickness of the modeled ear in mice to varying degrees.
(3) Effect on the weight of the modeled ear in animals: The compound of formula (I) (10, 20, 50 mg/kg) is administered by oral gavage twice daily for 8 consecutive days, which can significantly reduce the weight of the modeled ear in animals, and all three dose groups exhibit a good dose-response relationship in reducing the weight of the modeled ear in animals.
(4) Effect on the total score of the modeled ear in animals: The compound of formula (I) (10, 20, 50 mg/kg) is administered by oral gavage twice daily for 8 consecutive days, which can significantly reduce the total score of the affected ear in mice from D5 to D9, demonstrating good efficacy and exhibiting a good dose-response relationship.

## Claims

1. A method for preparing a compound of formula (I), 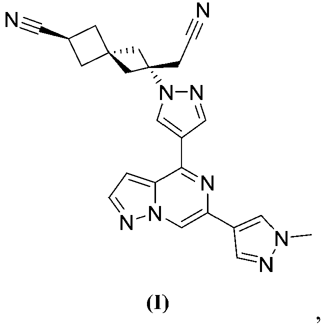 wherein the method comprises a chiral resolution method for compound q, 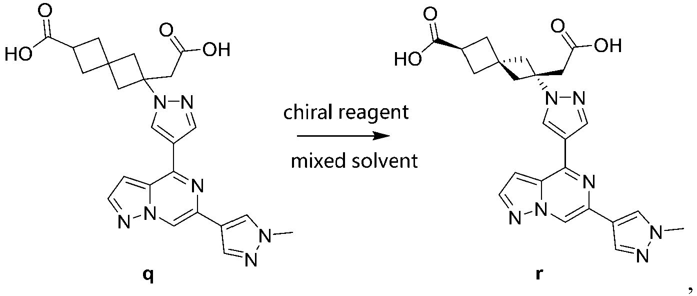 wherein
the chiral reagent is selected from the group consisting of chiral reagent 1 and chiral reagent 2;
the chiral reagent 1 is (*R*)-(+)-*N*-benzyl-1-phenylethylamine, and the chiral reagent 2 is (*S*)-(-)-*N*-benzyl-1-phenylethylamine;
the mixed solvent is selected from the group consisting of solvent 2 and solvent 3;
the solvent 2 is tetrahydrofuran, and the solvent 3 is water.

2. The method for preparing the compound of formula (I) according to claim 1, wherein the molar ratio of compound q to the chiral reagent is 1:2 to 1:2.5, preferably 1:2.1.

3. The method for preparing the compound of formula (I) according to claim 1, wherein the volume ratio of the mixed solvent is tetrahydrofuran: water = 15:1 to 20:1; preferably 16.5:1.

4. The method for preparing the compound of formula (I) according to claim 1, wherein the chiral resolution method for compound q further comprises a step of further purification.

5. The method for preparing the compound of formula (I) according to claim 1, comprising performing a selective alkylation method between compound d and compound e, 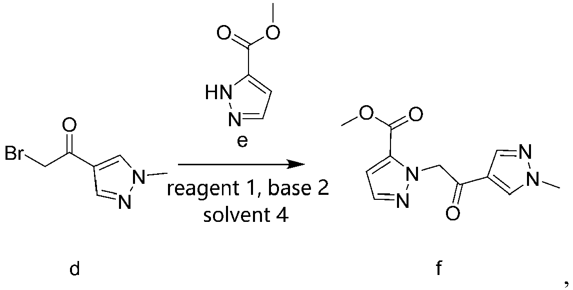 wherein
the reagent 1 is magnesium bromide;
the base 2 is DIEA;
the solvent 4 is tetrahydrofuran.

6. The method for preparing the compound of formula (I) according to claim 5, wherein the molar ratio of compound e, compound d, and the reagent 1 is 1:0.8:0.1 to 1:1.2:0.3, preferably 1:1:0.2.

7. The method for preparing the compound of formula (I) according to claim 1, comprising a method for synthesizing intermediate k, 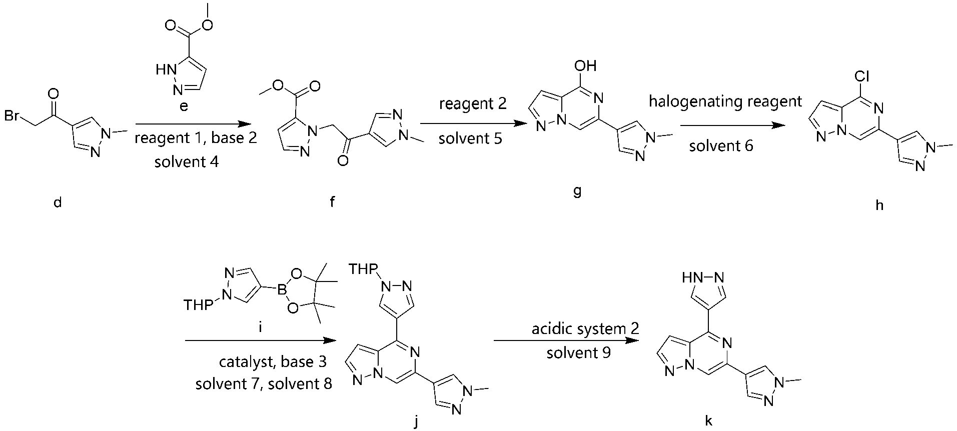
the reagent 1 is magnesium bromide;
the base 2 is DIEA;
the solvent 4 is tetrahydrofuran;
the reagent 2 is ammonium acetate;
the solvent 5 is ethanol;
the halogenating reagent is phosphorus oxychloride;
the solvent 6 is acetonitrile;
the catalyst is [1,1'-bis(diphenylphosphino)ferrocene]dichloropalladium(II);
the base 3 is sodium carbonate;
the solvent 7 is dioxane;
the solvent 8 is water;
the acidic system 2 is a hydrogen chloride/ethyl acetate solution;
the solvent 9 is dichloromethane.

8. The method for preparing the compound of formula (I) according to claim 1, further comprising a method for synthesizing the compound of formula (I) from compound 1, 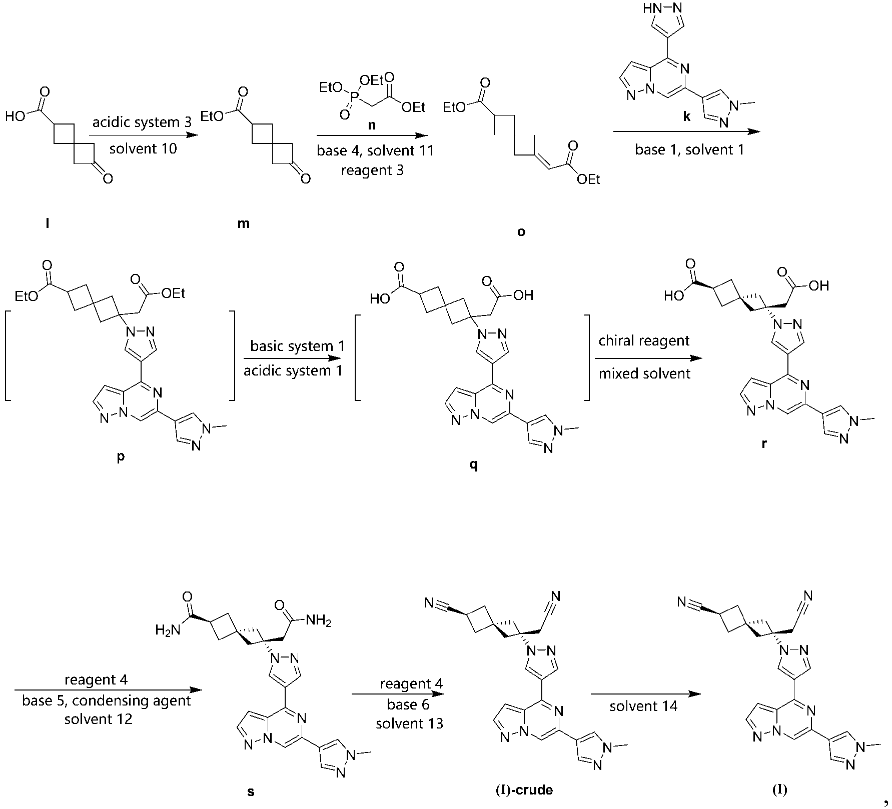
the acidic system 3 is sulfuric acid;
the solvent 10 is ethanol;
the base 4 is triethylamine;
the solvent 11 is tetrahydrofuran;
the reagent 3 is lithium bromide;
the solvent 1 is acetonitrile;
the basic system 1 is an aqueous sodium hydroxide solution;
the acidic system 1 is concentrated hydrochloric acid;
the chiral reagent is selected from the group consisting of chiral reagent 1 and chiral reagent 2;
the chiral reagent 1 is (*R*)-(+)-*N*-benzyl-1-phenylethylamine, and the chiral reagent 2 is (*S*)-(-)-*N*-benzyl-1-phenylethylamine;
the mixed solvent is selected from the group consisting of solvent 2 and solvent 3;
the solvent 2 is tetrahydrofuran, and the solvent 3 is water;
the reagent 4 is ammonium chloride;
the base 5 is DIEA;
the condensing agent is HATU;
the solvent 12 is DMF;
the reagent 4 is trifluoroacetic anhydride;
the base 6 is triethylamine;
the solvent 13 is dichloromethane;
the solvent 14 is methanol.

9. A Form 1 crystal form of a compound of formula (I) as shown below, 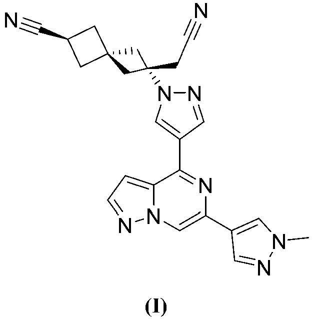 wherein the Form 1 crystal form of the compound of formula (I) has an X-ray powder diffraction pattern, measured using Cu Kα radiation, comprising characteristic diffraction peaks at the following 2θ angles: 8.087 ± 0.200°, 9.379 ± 0.200°, 11.188 ± 0.200°, and 12.852 ± 0.200°.

10. The Form 1 crystal form of the compound of formula (I) according to claim 9, wherein the Form 1 crystal form of the compound of formula (I) has an X-ray powder diffraction pattern, expressed in terms of 2θ angles, comprising at least 6, 7, 8, or 9 diffraction peaks selected from the group consisting of: 8.087 ± 0.200°, 9.379 ± 0.200°, 11.188 ± 0.200°, 12.852 ± 0.200°, 16.163 ± 0.200°, 18.306 ± 0.200°, 18.626 ± 0.200°, 19.105 ± 0.200°, 19.447 ± 0.200°, and 20.666 ± 0.200°.

11. The Form 1 crystal form of the compound of formula (I) according to claim 10, wherein the Form 1 crystal form of the compound of formula (I) has an X-ray powder diffraction pattern comprising characteristic diffraction peaks at the following 2θ angles: 8.087 ± 0.200°, 9.379 ± 0.200°, 11.188 ± 0.200°, 12.852 ± 0.200°, 16.163 ± 0.200°, 18.306 ± 0.200°, 18.626 ± 0.200°, and 19.105 ± 0.200°.

12. The Form 1 crystal form of the compound of formula (I) according to claim 11, wherein the Form 1 crystal form of the compound of formula (I) has an X-ray powder diffraction pattern comprising characteristic diffraction peaks at the following 20 angles: 8.087 ± 0.200°, 9.379 ± 0.200°, 11.188 ± 0.200°, 12.852 ± 0.200°, 16.163 ± 0.200°, 18.306 ± 0.200°, 18.626 ± 0.200°, 19.105 ± 0.200°, 19.447 ± 0.200°, 20.666 ± 0.200°, 20.875 ± 0.200°, and 21.210 ± 0.200°.

13. The Form 1 crystal form of the compound of formula (I) according to claim 12, wherein the Form 1 crystal form of the compound of formula (I) has an X-ray powder diffraction pattern comprising characteristic diffraction peaks at the following 20 angles: 8.087 ± 0.200°, 9.379 ± 0.200°, 11.188 ± 0.200°, 12.852 ± 0.200°, 16.163 ± 0.200°, 18.306 ± 0.200°, 18.626 ± 0.200°, 19.105 ± 0.200°, 19.447 ± 0.200°, 20.666 ± 0.200°, 20.875 ± 0.200°, 21.210 ± 0.200°, 22.417 ± 0.200°, 23.446 ± 0.200°, 24.043 ± 0.200°, and 24.402 ± 0.200°.

14. The Form 1 crystal form of the compound of formula (I) according to claim 13, wherein the Form 1 crystal form of the compound of formula (I) has an X-ray powder diffraction pattern comprising characteristic diffraction peaks at the following 20 angles: 6.516°, 8.087°, 9.378°, 11.188°, 11.881°, 12.852°, 13.880°, 14.564°, 15.615°, 16.163°, 18.306°, 18.626°, 19.105°, 19.477°, 20.666°, 20.875°, 21.210°, 22.417°, 23.446°, 23.715°, 24.043°, 24.402°, 25.159°, 25.663°, 26.227°, 26.742°, 26.897°, 27.127°, 27.752°, 28.341°, 29.332°, 30.727°, 30.956°, 31.678°, 32.197°, 32.574°, 34.479°, 35.051°, 35.341°, 35.873°, and 36.960°.

15. The Form 1 crystal form of the compound of formula (I) according to claim 9, wherein the Form 1 crystal form of the compound of formula (I) has an XRPD pattern as shown in FIG. 1.

16. The Form 1 crystal form of the compound of formula (I) according to claim 9, wherein the Form 1 crystal form of the compound of formula (I) has a differential scanning calorimetry (DSC) curve showing an endothermic peak onset at 161.77°C ± 3.00°C.

17. The Form 1 crystal form of the compound of formula (I) according to claim 9, wherein the Form 1 crystal form of the compound of formula (I) has a thermogravimetric analysis (TGA) curve showing a weight loss of 0.3182% at 150.00°C ± 3.00°C.

18. A use of the Form 1 crystal form of the compound of formula (I) as defined in any one of claims 9 to 17 in the manufacture of a medicament for treating psoriasis and/or inflammatory bowel disease.
